# EUROPEAN PATENT APPLICATION

(11) **EP 4 227 316 A1**
(43) Date of publication of application: **16.08.2023**
(21) Application number: 21878034.4
(22) Date of filing: 07.10.2021
(51) Int. Cl.: C07K 14/705, C07K 16/28, C12N 5/0783

(54) **T CELL RECEPTOR, IMMUNE CELL COMPRISING T CELL RECEPTOR, AND METHOD USING SAME**

(30) Priority: 07.10.2020 KR 20200129446
(71) Applicant: Daan Biotherapeutics Co., Ltd., Seoul 03766 (KR)
(72) Inventor: CHO, Byoung Chul, Seoul 04425 (KR); PYO, Kyoung Ho, Paju-si, Gyeonggi-do 10909 (KR); KIM, Jae Hwan, Seoul 06998 (KR); BYEON, Yeong Seon, Seoul 03462 (KR); KIM, Young Seob, Hwaseong-si, Gyeonggi-do 18423 (KR); XIN, Chun Feng, Seoul 05066 (KR)
(74) Representative: AWA Sweden AB
(86) International application number: PCT/KR2021/013826
(87) International publication number: WO 2022/075784

(57) **Abstract**

The present specification provides not only an antigen-specific T cell receptor but also a nucleic acid, a vector, and an immune cell comprising the T cell receptor, and a method using same.

## Description

### [Technical Field]

The present invention relates to a T cell receptor, an NK cell including a T cell receptor, and a method of using the same.

### [Background Art]

As of 2018, cell therapies for cancer treatment have been already used for cancer treatment in the market with Axicabtagene ciloleucel, Tisagenlecleucel, Immuncell-LC, etc. which are blood cancer therapies developed by Gilead, Novartis, and MOLMED, and cell therapies that have entered phase 2/3 are also undergoing clinical trials. More than 50% of the development of total cell therapies has been conducted in North America (USA) (344 cases as of 2018), and a significant portion of research is also being conducted in China (203 cases).

Various types of cell therapies, such as CAR-T suitable for recognizing and activating the surface of cancer cells, TCR-T modified with TCR, autologous T cells recognizing tumor antigens, and TIL, have been developed and are under preclinical and clinical studies. In particular, cell therapies targeting CD 19 have been developed the most, followed by TAA/TSA, BCMA, GD2, HER2, and the like. CD19 is almost only a cell therapy that has entered the market, and an IND process is currently conducted in association with most clinical and preclinical studies.

When comparing cell therapies with currently used immune check point inhibitors, there are limitations for immunotherapies in that the efficacy of immune checkpoint inhibitors is not sufficient due to the lack of T cells in the tumor and that among the T cells in the tumor, T cells targeting tumor antigens are less than 5%. Therefore, cell therapies that can intensively attack tumors have an advantage of proliferating and utilizing only immune cells targeting tumors.

Lung cancer has a high mortality rate worldwide, especially among Asian lung cancer patients, EGFR mutant tumors are quite high at 50%, and 27% thereof have mutations for L858R. The characteristic of EGFR mutation is that the effect of an EGFR-targeted therapy is exhibited well at the beginning, but after 8 to 10 months, most patients have acquired tolerance, and there is no therapy after acquired tolerance. In particular, as it is known that there is no effect on a combined treatment with immune checkpoint inhibitors, it is necessary to develop new therapies.

Thus, there is a need for a therapy that specifically targets a critical genetic damage that indicates the growth of these tumors.

The background art of the invention has been prepared to further facilitate understanding of the present invention. It should not be understood that the matters described in the background art of the invention exist as prior arts.

### [Disclosure]

### [Technical Problem]

Meanwhile, natural killer cells (NK cells) are cytotoxic lymphocytes constituting a major component of the innate immune system. The NK cells, which generally represent about 10 to 15% of circulating lymphocytes, are non-specific to antigens and may target and bind to many malignant cells including virus-infected cells without preceding immune sensitization, and furthermore, may kill the malignant cells. At this time, the death of the targeting cells may be caused by inducing cytolysis.

Accordingly, the NK cells are isolated from peripheral blood lymphocytes of a subject for therapeutic purposes, and the isolated NK cells are again used by a method in which large numbers are obtained through cell culture and then re-injected into the subject. Such NK cell treatment, that is, *ex vivo* and *in vivo* injection therapy, is in the limelight by inducing highly effective cell death in infected or tumor cells. However, the NK cell injection therapy has a limitation to being limited to targets.

Accordingly, the present inventors paid attention to NK cells through genetic modification in order to overcome the limitation. More specifically, conventional NK cell-based cell therapies establish strategies such as inducing antibody-dependent cytotoxic responses through the expression of Fc receptors or targeting cancer through the expression of chimeric antigen receptors. However, these strategies have not been largely effective in the treatment of solid tumor.

Accordingly, the present inventors paid attention to a T cell receptor specific to a specific antigen in order to overcome the limitations of the treatment of solid tumor described above, and through this, tried to enhance the targeting of NK cells to solid tumor.

Eventually, the present inventors identified a T cell receptor capable of recognizing a specific molecular mutation for solid tumor, and modified a gene to be expressed in NK cells (NK-92) and cell lines, thereby enhancing the targeting of NK cells to solid tumor.

Furthermore, the present inventors recognized that the immune response to the targeted solid tumor may be further improved when the Fc receptor was further included in the NK cells described above. Accordingly, the present inventors developed NK cells with improved targeting for solid tumor and an anticancer effect thereof by including both a T cell receptor and an Fc receptor.

More specifically, the present inventors identified an analysis platform showing an antigen prediction rate of 90% or more based on WES and RNA seq results of cancer patients. At this time, an algorithm applied to the analysis identified antigen candidates through Neopepsee based on a Net-MHC algorithm. Next, a process of validating the antigen was performed by synthesizing a predicted antigen and treating the antigen in DC and CD8 T cells.

Accordingly, major HLA-A alleles capable of recognizing an antigen for L858R were found. As a result, it may be predicted that the HVKITDFGR antigen for A*33:03 not only has high binding affinity, but also may act sufficiently as an antigen based on 10 or more parameters provided by Neopepsee.

Based on this, a validation platform capable of predicting a tumor-specific antigen was established to confirm a response to the antigen. The validation platform recognized antigens by differentiating dendritic cells (DCs) from peripheral blood mononuclear cells (PBMCs) of normal subjects matched with HLA-A, and based on this, the antigen response to L858R was measured.

As a result, as predicted above, T cells that responded specifically to tumors were produced in patients with HLA-A recognizing L858R, and the response to the antigen (IFN-gamma ELISPOT) was confirmed.

In addition, it was confirmed that the TCR of the T cells identified by the above-described process may be expressed in immune cells.

The present invention has been made in an effort to provide an EGFR-mutant-targeted cell therapy capable of directly killing cancer cells by providing T cells or NK-92 cells with a TCR targeting an MHC-1-L858R neoantigen complex that increases on the surface of cancer cells.

The present invention has also been made in an effort to provide a cell therapy with further improved killing function for cancer cells by further including an Fc receptor in the T cells or NK-92 cells having the above-described TCR to further improve an immune response thereto.

Furthermore, since the proportion of Asians in the aforementioned HLA-A is 20% or more, it is expected that significant patients with EGFR mutations will benefit from the present invention.

The objects of the present invention are not limited to the aforementioned objects, and other objects, which are not mentioned above, will be apparent to those skilled in the art from the following description.

### [Technical Solution]

According to an embodiment of the present invention, there is provided a T cell receptor including:
(i) a complementarity determining region (CDR) 3 of a T cell receptor (TCR) alpha chain variable region including an amino acid sequence CAFIGHGGSQGNLIF (SEQ ID NO: 1) or a variant thereof, and
   a CDR3 of a TCR beta chain variable region including an amino acid sequence CASSMQGAMSEQFF (SEQ ID NO: 13) or a variant thereof,
(ii) a CDR 3 of a TCR alpha chain variable region including an amino acid sequence CAATGTYKYIF (SEQ ID NO: 2) or a variant thereof, and
   a CDR 3 of a TCR beta chain variable region including an amino acid sequence CASSPEFARALDNQPQHF (SEQ ID NO: 14) or a variant thereof,
(iii) a CDR 3 of a TCR alpha chain variable region including an amino acid sequence CAYGGGSEKLVF (SEQ ID NO: 3) or a variant thereof, and
   a CDR 3 of a TCR beta chain variable region including an amino acid sequence CASSSATGTQGYTF (SEQ ID NO: 15) or a variant thereof,
(iv) a CDR 3 of a TCR alpha chain variable region including an amino acid sequence CALINARLMF (SEQ ID NO: 4) or a variant thereof, and
   a CDR 3 of a TCR beta chain variable region including an amino acid sequence CASSFTNTGELFF (SEQ ID NO: 16) or a variant thereof,
(v) a CDR 3 of a TCR alpha chain variable region including an amino acid sequence CAVNGGSQGNLIF (SEQ ID NO: 5) or a variant thereof, and
   a CDR 3 of a TCR beta chain variable region including an amino acid sequence CASSMWQGNGEQYF (SEQ ID NO: 17) or a variant thereof,
(vi) a CDR 3 of a TCR alpha chain variable region including an amino acid sequence CAMREGYGGATNKLIF (SEQ ID NO: 6) or a variant thereof, and
   a CDR 3 of a TCR beta chain variable region including an amino acid sequence CASSVGPGTTSYNEQFF (SEQ ID NO: 18) or a variant thereof,
(vii) a CDR 3 of a TCR alpha chain variable region including an amino acid sequence CAYNNGDGGSQGNLIF (SEQ ID NO: 7) or a variant thereof, and
   a CDR 3 of a TCR beta chain variable region including an amino acid sequence CATSRDRSTDTQYF (SEQ ID NO: 19) or a variant thereof,
(viii) a CDR 3 of a TCR alpha chain variable region including an amino acid sequence CATDGGSARQLTF (SEQ ID NO: 8) or a variant thereof, and
   a CDR 3 of a TCR beta chain variable region including an amino acid sequence CASSLGLSGYTF (SEQ ID NO: 20) or a variant thereof,
(ix) a CDR 3 of a TCR alpha chain variable region including an amino acid sequence CATLYNTDKLIF (SEQ ID NO: 9) or a variant thereof, and
   a CDR3 of a TCR beta chain variable region including an amino acid sequence CASSQSMNTEAFF (SEQ ID NO: 21) or a variant thereof,
(x) a CDR 3 of a TCR alpha chain variable region including an amino acid sequence CAMRGPWRGSSGSARQLTF (SEQ ID NO: 10) or a variant thereof, and
   a CDR 3 of a TCR beta chain variable region including an amino acid sequence CASRTGLSYEQYF (SEQ ID NO: 22) or a variant thereof,
(xi) a CDR 3 of a TCR alpha chain variable region including an amino acid sequence CALSVRGFKTSYDKVIF (SEQ ID NO: 11) or a variant thereof, and
   a CDR 3 of a TCR beta chain variable region including an amino acid sequence CASSFGSAYNEQFF (SEQ ID NO: 23) or a variant thereof, or
(xii) a CDR 3 of a TCR alpha chain variable region including an amino acid sequence CAVNMMDSSYKLIF (SEQ ID NO: 12) or a variant thereof, and
   a CDR 3 of a TCR beta chain variable region including an amino acid sequence CASSFPTARSNTEAFF (SEQ ID NO: 24) or a variant thereof.

According to a feature of the present invention, the T cell receptor may bind to an epitope included in an amino acid sequence of HVKITDFGR (SEQ ID NO: 49) or an MHC-binding form thereof, and the epitope may have a binding affinity with at least one of HLA-A*33:03 and HLA-A*31:01.

According to another feature of the present invention, the T cell receptor may target an EGFR L858R mutation.

According to yet another feature of the present invention, the TCR alpha chain variable region may consist of an amino acid sequence represented by SEQ ID NO: 55, and the SEQ ID NO: 55 may include an amino acid sequence represented by SEQ ID NO: 1.

According to yet another feature of the present invention, the TCR alpha chain variable region may consist of an amino acid sequence represented by SEQ ID NO: 57, and the SEQ ID NO: 57 may include an amino acid sequence represented by SEQ ID NO: 2.

According to yet another feature of the present invention, the TCR beta chain variable region may consist of an amino acid sequence represented by SEQ ID NO: 56, and the SEQ ID NO: 56 may include an amino acid sequence represented by SEQ ID NO: 13.

According to yet another feature of the present invention, the TCR beta chain variable region may consist of an amino acid sequence represented by SEQ ID NO: 58, and the SEQ ID NO: 58 may include an amino acid sequence represented by SEQ ID NO: 14.

According to yet another feature of the present invention, the T cell receptor may be a single chain type, but is not limited thereto.

According to yet another feature of the present invention, the TCR alpha chain variable region and the TCR beta chain variable region may be linked to each other by a linker sequence.

According to another aspect of the present invention, there is provided a nucleic acid encoding the T cell receptor according to the aforementioned contents.

According to a feature of the present invention, the nucleic acid may include at least one nucleic acid sequence represented by SEQ ID NOs: 51 to 54; or a nucleic acid sequence having at least 80% or more identity with at least one nucleic acid sequence represented by SEQ ID NOs: 51 to 54.

At this time, the SEQ ID NOs: 51 and 53 may refer to nucleic acid sequences encoding the TCR alpha chain variable region and the SEQ ID NOs: 52 and 54 may refer to nucleic acid sequences encoding the TCR beta chain variable region.

According to another feature of the present invention, the nucleic acid may further include Furin, 2A, and IRES sequences, but is not limited thereto.

According to yet another aspect of the present invention, there is provided a vector including the nucleic acid according to the aforementioned contents.

At this time, the vector may be an expression vector and may be a lentiviral vector, but is not limited thereto, and may include all expression vectors that may be used in the art.

According to a feature of the present invention, the vector may include a nucleic acid sequence represented by SEQ ID NO: 50; or a nucleic acid sequence having at least 80% or more identity with the nucleic acid sequence represented by SEQ ID NO: 50.

According to still another aspect of the present invention, there is provided an immune cell including the T cell receptor according to the aforementioned contents.

At this time, the immune cell may include a TCR, a nucleic acid, and a vector according to the aforementioned contents, and may be an NK-92 cell. That is, the immune cell may be NK-92 expressed with the TCR by including the TCR, the nucleic acid, and the vector according to the aforementioned contents.

Furthermore, the immune cell may be an NK-92 cell, but is not limited thereto, and may include all various immune cells which may be modified by inserting the TCR.

According to still yet another aspect of the present invention, there is provided a cell therapy including the immune cell including the T cell receptor.

According to a feature of the present invention, the cell therapy may further include an effector T cell.

According to another feature of the present invention, the cell therapy may be a solid tumor therapy, but is not limited thereto, and may be a therapy for all carcinomas including an HVKITDFGR antigen represented by SEQ ID NO: 49 according to an embodiment of the present invention.

Hereinafter, the present invention will be described in more detail with reference to Examples. However, these Examples are only illustrative of the present invention, and the scope of the present invention is not limited to these Examples.

### [Advantageous Effects]

The present invention can be proposed as a new therapy for tumors with acquired tolerance. More specifically, conventional lung cancer therapies are effective initially, but after 8 to 10 months, acquired tolerance occurs in most patients. However, there is no alternative therapy therefor.

Accordingly, the present invention can be proposed as a new therapy capable of overcoming these limitations. In addition, the present invention has an effect of targeting and treating harboring EGFR by mutations, as well as acquired tolerance.

Furthermore, unlike drugs having a half-life, the present invention has an effect of increasing a killing effect on tumors by increasing other immune responses by initial activation of T cells.

More specifically, the present invention induces the differentiation and proliferation of memory T cells to avoid the defense mechanisms of cancer cells, thereby treating cancer or tumor conditions and preventing recurrence, progression, and metastasis.

In addition, the present invention has an effect of increasing an effect of each drug in parallel with conventional therapies such as TKI and PD-1/PD-L1, thereby increasing the survival rate of patients.

The effects of the present invention are not limited by the foregoing, and other various effects are anticipated herein.

### [Description of Drawings]

FIG. 1 illustrates a system for predicting an antigen of a cancer patient and a method for preparing TCR-T or TCR-NK cells based on the system.
FIG. 2 illustrates an example of a process for verifying an antigen predicted with Neopepsee.
FIG. 3 illustrates validation results of antigen-specific T cells analyzed by a validation platform.
FIGS. 4A and 4B illustrate results of HLA-A and antigen sequences targeting L858R.
FIGS. 5A to 5D illustrate results of tumor-specific T cells specifically adhering to an EGFR-MT-L858R-HVKITDFGR-A*33:03 tetramer.
FIG. 6 illustrates a result of cytolytic factors of T cells with activity specifically induced to an antigen (EGFR-MT-L858R-HVKITDFGR-A*33:03 tetramer).
FIG. 7 illustrates a distribution diagram of T cell receptors with activity specifically induced to an antigen (EGFR-MT-L858R-HVKITDFGR-A*33:03 tetramer).
FIG. 8 illustrates a result of an NK-92 cell line when cultured in a T flask.
FIG. 9 illustrates a result of the proliferation efficiency of NK-92 according to concentrations of FBS and IL-2.
FIG. 10 illustrates results of the cell proliferation efficiency of an NK-92 cell line according to a culture method.
FIGS. 11A and 11B illustrate results of an NK-92 cell line and activating factors and toxic factors of the NK-92 cell line expressing an Fc receptor according to a culture condition.
FIG. 12 illustrates results of the GFP expression efficiency of NK-92 cells according to a protein expression promoter.
FIGS. 13A and 13B illustrate results of the expression of chemokine receptors in NK-92 cells.
FIGS. 14A and 14B illustrate results of the expression of CD3 molecules and T cell receptor molecules in NK-92 cells.
FIG. 15A is a lentiviral vector map for expressing a TCR sequence according to an embodiment of the present invention.
FIG. 15B is a schematic diagram of nucleic acid sequences of a CD3 molecule and a T cell receptor for expressing a TCR sequence according to an embodiment of the present invention.
FIGS. 16A and 16B illustrate results of confirming the cell activity of a TCR according to an embodiment of the present invention.
FIGS. 17A and 17B illustrate results of confirming a cytolytic effect on NK cells including a TCR according to an embodiment of the present invention.
FIG. 18 is a result of confirming a tumor suppression effect of NK cells including the TCR according to an embodiment of the present invention.

### [Modes of the Invention]

Advantages and features of the present invention, and methods for accomplishing the same will be more clearly understood from embodiments to be described below in detail with reference to the accompanying drawings. However, the present invention is not limited to the embodiments set forth below, and will be embodied in various different forms. The embodiments are just for rendering the disclosure of the present invention complete and are set forth to provide a complete understanding of the scope of the invention to a person with ordinary skill in the art to which the present invention pertains, and the present invention will only be defined by the scope of the claims. As used herein, the term "T-cell receptor (TCR)" includes not only a natural TCR, but also TCR variants, fragments, and constructs. Thus, the TCR includes a multimer and a single chain structure as well as a heterodimer including TCR alpha and beta chains by optionally including additional domains and/or moieties.

As used herein, the term "epitope" generally refers to a site on an antigen recognized by a binding domain, typically a (poly-)peptide. At this time, the binding domain means an antigen binding site. That is, the binding domain means a domain of a molecule that binds to and interacts with a specific epitope of an antigenic target.

As used herein, the term "natural killer cell (NK cell)" is a cell of the immune system that kills target cells in the absence of specific antigenic stimulation, without restriction according to a major histocompatibility complex (MHC) class. The target cells may be cancer or tumor cells. The NK cells are characterized by the presence of CD56 and the absence of a CD3 surface marker. Therefore, research will be conducted by artificially inserting CD3-gamma, delta, zeta, etc.

As used herein, the term "vector" refers to a non-chromosomal nucleic acid containing an intact replicon so that the vector may replicate when located in a permissive cell by a transformation process. The vector may replicate in bacteria, but may have limited replication capacity in mammalian cells, and may be viral or non-viral. The non-viral vector for delivering the nucleic acid includes naked DNA, DNA complexed with cationic lipids alone or in combination with a cationic polymer, anionic and cationic liposomes, a cationic polymer contained in liposomes, heterologous polylysine, oligopeptide of defined length, a DNA protein complex including DNA condensed with polyethyleneamine, and a ternary complex including particles, virus, and polylysine DNA.

As used herein, the term "immune cell" may include T cells, natural killer T cells (NKT), natural killer cells (NK), human embryonic stem cells, and hematopoietic stem cells (HSCs) and induced pluripotent stem cells (iPSs). At this time, the T cells may be cytotoxic T cells (CTL), regulatory T lymphocytes, inflammatory T lymphocytes, helper T lymphocytes and gamma-delta T cells, and innate lymphocytes (ILC1, ILC2), and further, the above-mentioned T cells may be CD4+, CD8+, and mixed populations thereof.

As used herein, the term "human leukocyte antigen (HLA)" refers to a human gene encoding a major histocompatibility complex (MHC) protein on the surface of a cell responsible for regulating the immune system.

As used herein, the term "dendritic cells (DCs)" refer to members of a diverse population of morphologically similar cell types found in lymphoid or non-lymphoid tissues. These cells are characterized by their unique morphology and high expression levels of surface MHC class I and II molecules, which are proteins that present antigenic peptides to T cells. APCs (macrophages, etc.) including DCs and T cells may be isolated or derived and differentiated from peripheral blood and many tissues conveniently, such as peripheral blood mononuclear cells (PBMCs) derived from the peripheral blood.

As used herein, the term "treatment" may include any activity that improves or benefits cancer symptoms without limitation.

As used herein, the term "specific(ally) binding" generally means that the TCR binds to its desired antigenic target more easily than a randomly unrelated non-target antigen through its antigen binding site. Furthermore, the "specifically binding" may mean that binding specificity of the TCR to its antigen target may be at least about 5 times, preferably 10 times, more preferably 25 times, even more preferably 50 times, and most preferably at least 100 times larger than its binding specificity to a non-target antigen.

### Antigen prediction and validation process

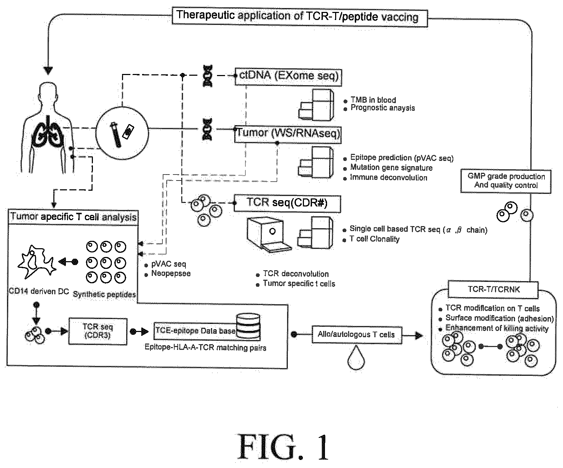

Hereinafter, an antigen prediction and validation process through Neopepsee will be described with reference to FIGS. 1 to 3B.

FIG. 1 illustrates a system for predicting an antigen of a cancer patient and a method for preparing TCR-T or TCR-NK cells based on the system. For example, after somatic mutations in patient tumors are obtained from patient's tumor through RNA sequencing, whole exome sequencing, and reference WES in normal blood, monocytes derived from patients are differentiated into dendritic cells, T cells are proliferated through antigen presentation by exposing an epitope identified from the previously identified mutant gene, the reactivity of the proliferated T cells is confirmed, a CDR3 sequence of the TCR is obtained through TCR sequencing of T cells based on the confirmed reaction, and then T cells are modified into tumor-specific cells through a gene delivery carrier containing this sequence. Therefore, it is possible to generate and validate cell therapies based on autologous T cells or NK-92 cells.

More specifically, first, antigen prediction should be preceded the earliest to develop a vaccine for tumor or a cell therapy. The antigen prediction is validated based on the expression of an abnormal mutant protein obtained by whole exome sequencing (WES) for tumors, and a mutant antigen through RNA seq. When the affinity between the tumor antigen and MHC-1 increases, it may mean that there is a potential as an antigen that can be recognized by immune cells. In the study, Neopepsee was used as an analysis pipeline for antigen analysis.

Thereafter, the peptide for the predicted antigen is synthesized/purified with 9 amino acids at high purity of 95% or more, and exposed to dendritic cells with LPS using DMSO as a solvent, which is the most used to dissolve the antigen to be differentiated into mature DC, and then subjected to education of T cells.

Then, validation according to the expression level of IFN-gamma is performed.

As an existing validation platform capable of analyzing antigens, pVAC seq is most commonly used, and an open analysis platform based on Linux and Python programs. As a central algorithm of this program, the binding affinity of HLA-A, called NetMHC-pan, may be quantified, and pVAC seq additionally includes the expression level of tumor antigens through RNA seq. The Neopepsee also includes the same algorithm and includes 12 additional parameters to show a more advanced analysis platform. The Neopepsee is an analysis platform that has secured superior results even in benchmarking analysis between previously reported antigen search programs. The Neopepsee is an open source program and an analysis technology that has recently been published in an academic journal.

Therefore, the present research team conducted an experiment by determining the presence or absence of mutations in L858R and E19Del based on patients with mutations in EGFR.

Referring to FIG. 1, first, analysis was performed using matched normal blood as a reference for WES in the patient's tumor, and germline mutation or SNV in WES. A tool used to determine the presence or absence of mutations in WES was a GATK2 pipeline, and analysis was performed based on mapping data for hg38.

50 ml of a patient's blood was obtained, and based on the IFNg ELISPOT data, the recognition rate of the antigen was validated through T cells in the DC present in the blood.

Then, when the validation of a candidate antigen presented in the computer was completed, the TCR seq of the T cells was analyzed to validate the major TCR capable of best recognizing the tumor antigen.

Next, a recombinant TCR for CDR3 was constructed through the TCR seq of the validated T cells to produce a cell therapy capable of specifically recognizing an antigen in autologous/allogeneic T cells and NK-92 cells and validate the cell therapy.

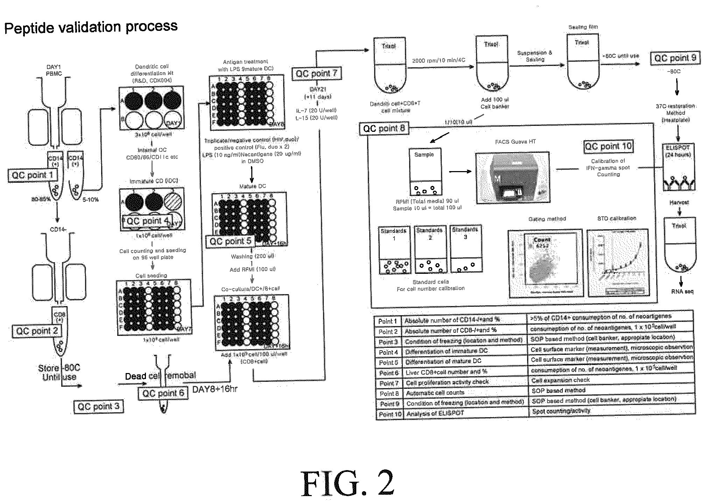

FIG. 2 illustrates an example of a process for validating an antigen predicted with Neopepsee. At this time, the entire antigen validation process was required for about 21 days, which was a platform capable of validating antigens that respond specifically to tumors from the patient's PBMC in a fairly short time. The antigen predicted with Neopepsee was differentiated into mDC together with a 9-mer peptide antigen synthesized from iDC differentiated from CD14, and the differentiated DC was co-cultured with T cells, and then proliferated into antigen-specific T cells, and validated based on the expression level of IFN-gamma. In each step, 10 important steps in the development of cell therapies were indicated and QC points were designated.

More specifically, referring to FIG. 2, first, on the first day, PBMCs were isolated from the patient's blood. At this time, a protocol used to isolate PBMCs was a method that can obtain lymphocytes and monocytes in the blood, and CD14-positive cells, that is, monocytes may be obtained through a magnet and an antibody-microbead method.

Then, CD14+ cells were subjected to a process of differentiating into DCs. At this time, differentiation into imDC was induced using GM-CSF, and IL-4.

Next, CD8+ cells were also taken from CD14- in the same manner, stored at - 80°C, and then used for co-culture on imDC capable of presenting antigens.

Then, on 7 and 8 days, the cells were treated with LPS together with 10 ug/ml of the previously synthesized tumor antigen and cultured for 16 hours to sufficiently recognize the tumor antigen in the DC.

Then, the cells were subjected to a washing process and cultured with CD8-positive cells.

Then, the DC-CD8+ cells were co-cultured for about 11 days, and cultured in dedicated media treated with IL-7/IL-15.

Then, cell counting of the T cells obtained after 11 days was performed, and after the cell counting was completed, the antigen was re-treated and the expression of IFN-gamma expressed in T cells was analyzed through ELISPOT.

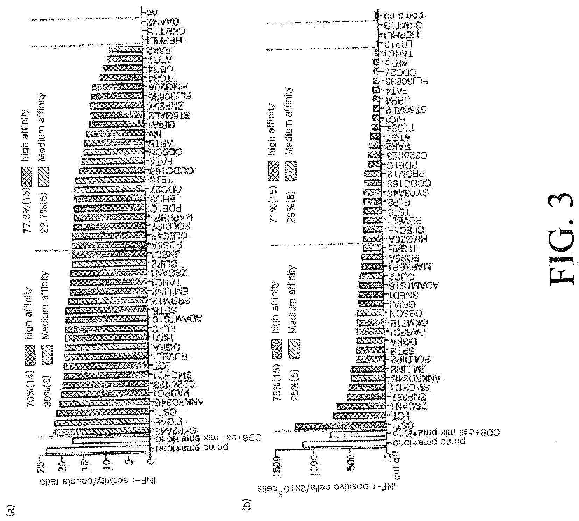

FIG. 3 illustrates results of validating antigen-specific T cells analyzed by a validation platform. At this time, to confirm the antigen validation ability for Neopepsee, after representative antigens of 20 patients (HLA-A type is A*:24:02 patients) were recognized in dendritic cells and T cells were educated, ELISPOT validation experiments were conducted to confirm whether there was actually a response to the tumor antigen. As a result, it was confirmed that there was an antigen validation ability of 90% or more.

More specifically, the experiment was conducted with A*24.02, which is the HLA-A type that accounts for the largest proportion worldwide. For 20 patients, specific antigens for each patient were predicted with Neopepsee, and as results of recognizing a total of 41 antigens in DC, educating T cells, and then checking the degree of response to these antigens, there was a response to each tumor antigen except for 3 of the 41 antigens. Furthermore, the response to each tumor antigen may be represented by the number and surface area (activity) of spots of IFN-gamma for the antigen, and the higher the number of spots of IFN-gamma, the better the presentation of the antigen, and the surface area may mean the amount of IFN-gamma for the antigen. Furthermore, it was determined as positive by cutting-off the IFN-gamma expression level of T cells in a non-treated group.

Accordingly, referring to FIG. 3, as predicted by Neopepsee, the expression prediction of IFN-gamma for the antigen is shown to have a predictive rate of 90% or more based on the expression of IFN-gamma. For 20 patients, specific antigens for each patient were predicted with Neopepsee, and as a result of predicting the response to 41 antigens, the response to the tumor antigen was confirmed by 90% or more.

As the result above, it is possible to select a tumor-specific antigen with high predictive ability, and to develop and verify cell therapies based thereon.

### Obtaining of specific antigens for EGFR mutant tumors

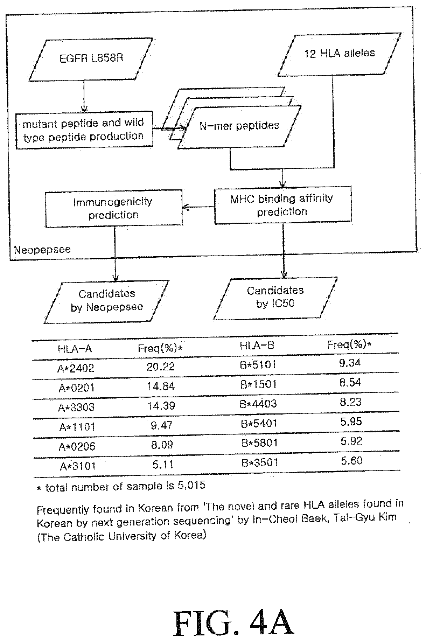

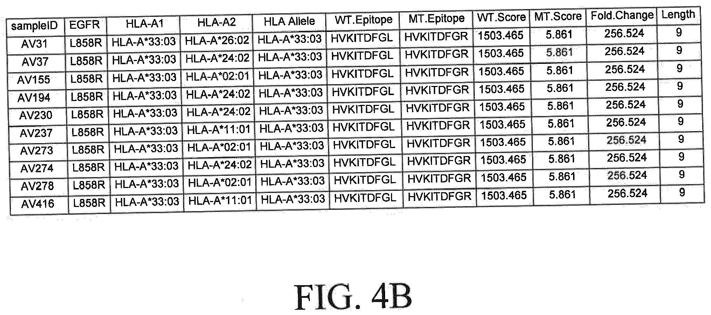

Hereinafter, specific antigens for EGFR mutant tumors will be described with reference to FIGS. 4A and 4B.

FIGS. 4A and 4B illustrate results of HLA-A and antigen sequences targeting L858R.

Referring to FIG. 4A, an antigen capable of recognizing an L858R mutation for EGFR was selected through Neopepsee, an L858R mutant peptide for EGFR and a wild type (WT) peptide were produced, and antigens capable of recognizing the L858R mutation for EGFR were applied to 12 major HLA-As, and the degree of antigen prediction for 9 to 11 mer was validated through MHC binding affinity.

By the process described above, HLA-A including HLA-A*2402, HLA-A*0201, HLA-A*3303, HLA-A^{∗}1101, HLA-A*0206, and HLA-A*3101 was selected, all of these had a high frequency of 5% or more, and among them, HLA-A*2402, HLA-A*0201, and HLA-A*3303 having a frequency of 10% or more were selected to derive antigens from a total of 10 patient samples including the L858R mutation for EGFR.

Accordingly, referring to FIG. 4B, the total of 10 patient samples containing the L858R mutation for EGFR commonly included HLA-A*3303, unlike HLA-A*2402 and HLA-A*0201, and then, HLA-A*3303 was selected to identify an antigen therefor.

Eventually, as the antigen of the HVKITDFGR sequence was shown to have the same response (result) in the patient samples based on HLA-A*3303, the target antigen for the L858R mutation to EGFR was selected as HVKITDFGR.

As the result above, it is possible to select a tumor-specific antigen with high predictive ability, and to develop and verify cell therapies based thereon.

### T cell receptor (TCR)

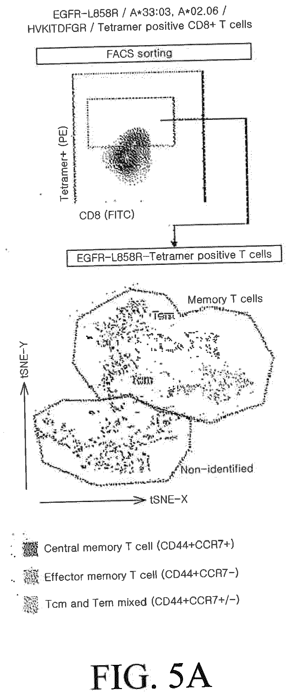

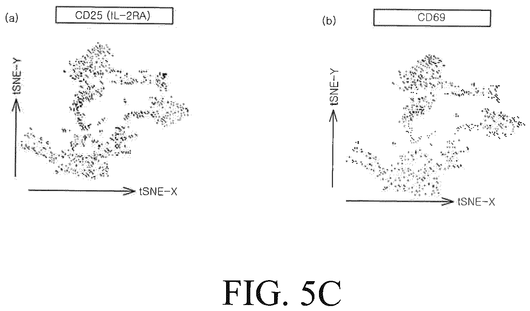

Hereinafter, referring to FIGS. 5A to 7, TCRs capable of targeting specific antigens to EGFR mutant tumors will be described. At this time, in order to identify a TCR capable of targeting a specific antigen for EGFR mutant tumor, EGFR-L858R-specific T cells were produced, and the produced T cells were sorted using a tetramer. Further, the isolated cells were analyzed through single cell RNA analysis technique.

More specifically, first, CD14+ positive cells were isolated from the blood of a subject, cultured with an EGFR-MT antigen (9mer) to induce antigen presentation, and then co-cultured with CD8+ positive cells of the same subject for 14 days, and thereafter, the presence of EGFR-L858R-specific T cells was confirmed through ELISPOT. Furthermore, after sorting T cells capable of binding to an EGFR-MT 9mer-MHC-1 complex using the tetramer, sequences for TCRs thereof were obtained through scRNA seq/VDJ.

First, referring to FIGS. 5A to 5D, a sorting process of T cells specific to the EGFR L858R mutant antigens will be described.

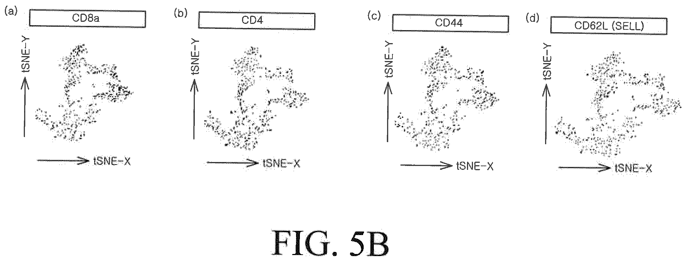

FIG. 5A is a result of tumor-specific T cells specifically adhering to an EGFR-MT-L858R-HVKITDFGR-A*33:03 tetramer. At this time, for convenience of description, the process will be described with reference to FIGS. 5B to 5D. Furthermore, samples were collected from subjects containing A*33:03/A*02.06 for identification of the TCR.

Tumor-specific T cells that specifically adhere to the EGFR-MT-L858R-HVKITDFGR-A*33:03 tetramer (EGFR-L858R-A*33:03 tetramer positive T cells) are memory T cells, which include CD44+CCR7+ central memory T cells (Tern), CD44+CCR7- effector memory T cells (Tern), and Tcm and Tern mixed cells (CD44+CCR7+/-).

More specifically, referring to FIGS. 5B to 5D, the expression results of the cell activating factors in the T cells described in FIG. 1A are illustrated.

First, referring to FIG. 5B, tumor-specific T cells (EGFR-L858R-A*33:03 tetramer positive T cells) specifically adhering to the EGFR-MT-L858R-HVKITDFGR-A*33:03 tetramer express CD8a, CD4, CD44, and CD62L, which are mature T cell factors, and thus, it may mean that the tumor-specific T cells (EGFR-L858R-A*33:03 tetramer positive T cells) specifically adhering to the EGFR-MT-L858R-HVKITDFGR-A*33:03 tetramer are mature T cells.

Next, referring to 5C, tumor-specific T cells (EGFR-L858R-A*33:03 tetramer positive T cells) specifically adhering to the EGFR-MT-L858R-HVKITDFGR-A*33:03 tetramer express CD25 and CD69, which are cell activators, and thus, it may mean that the tumor-specific T cells (EGFR-L858R-A*33:03 tetramer positive T cells) specifically adhering to the EGFR-MT-L858R-HVKITDFGR-A*33:03 tetramer are activated effector T cells.

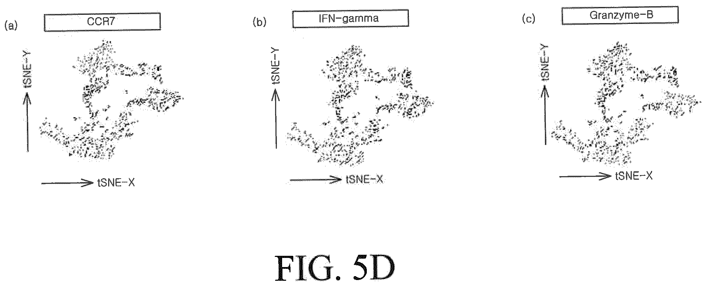

Next, referring to FIG. 5D, tumor-specific T cells (EGFR-L858R-A*33:03 tetramer positive T cells) specifically adhering to the EGFR-MT-L858R-HVKITDFGR-A*33:03 tetramer express CCR7, IFN-gamma, and Granzyme-B.

At this time, as CCR7 is a memory cell factor, the tumor-specific T cells (EGFR-L858R-A*33:03 tetramer positive T cells) specifically adhering to the EGFR-MT-L858R-HVKITDFGR-A*33:03 tetramer are memory T cells, which may survive *in vivo* for a long time and may cause a secondary immune response.

Furthermore, as IFN-gamma and Granzyme-B are cytotoxic factors, the tumor-specific T cells (EGFR-L858R-A*33:03 tetramer positive T cells) specifically adhering to the EGFR-MT-L858R-HVKITDFGR-A*33:03 tetramer may mean T cells capable of releasing cytokines.

Thus, the tumor-specific T cells (EGFR-L858R-A*33:03 tetramer positive T cells) specifically adhering to the EGFR-MT-L858R-HVKITDFGR-A*33:03 tetramer according to an embodiment of the present invention mean activated T cells in an *in vivo* immune response to a tumor containing the L858R mutation, and accordingly, may have an anticancer effect on a tumor containing the L858R mutation.

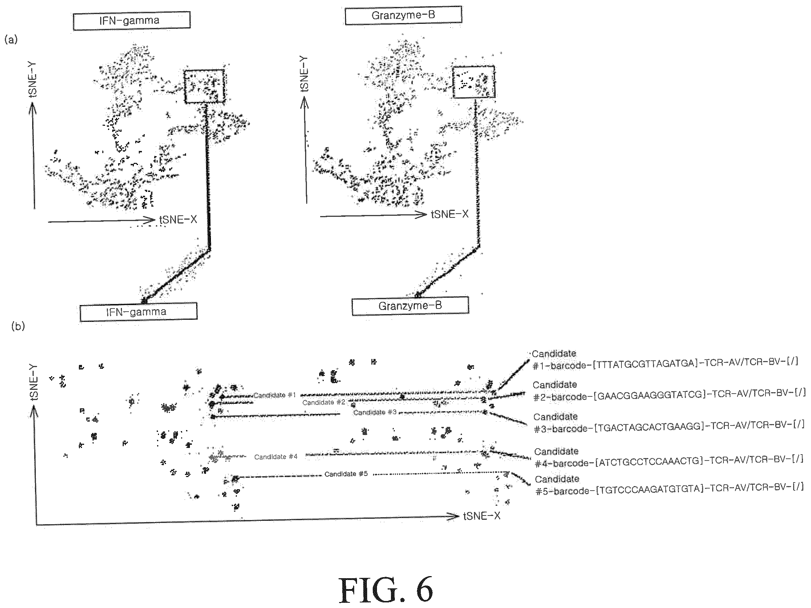

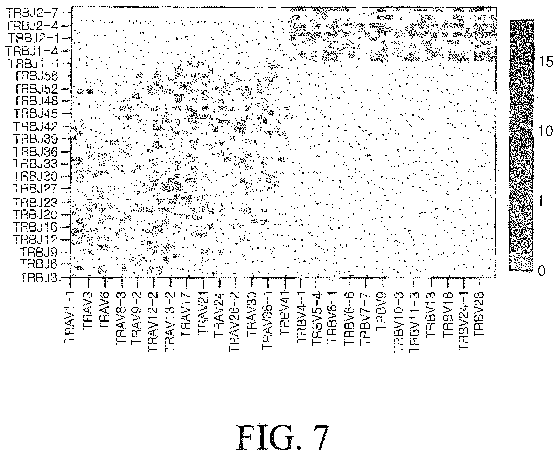

Hereinafter, the T cell receptor (TCR) specific to the EGFR L858R mutant antigen will be described with reference to FIGS. 6 and 7. At this time, the receptor sequence for T cells specific to the EGFR L858R mutant antigen was derived based on the expression of cytolytic factors in the selected T cells through the process of FIGS. 5A to 5D.

More specifically, FIG. 6 illustrates a result of a cytolytic factor of T cells with the activity specifically induced to an antigen (EGFR-MT-L858R-HVKITDFGR-A*33:03 tetramer).

First, referring to FIG. 6A, the cells expressing cytolytic factors among the tumor-specific T cells (EGFR-L858R-A*33:03 tetramer positive T cells) specifically adhering to the EGFR-MT-L858R-HVKITDFGR-A*33:03 tetramer account for about 10 to 15% of about 800 total cells, and account for about 27% of the total activated cells. That is, when the tumor-specific T cells (EGFR-L858R-A*33:03 tetramer positive T cells) specifically adhering to the EGFR-MT-L858R-HVKITDFGR-A*33:03 tetramer are activated, it may mean that a cytotoxic (cytolytic) factor may be expressed at a high rate, and accordingly, the T cells may have an effective cytotoxic (cytolytic) effect on tumors.

Accordingly, referring to FIG. 6B, only cells expressing the highest cytolytic factor among T cells expressing the aforementioned cytolytic factor were selected, and then a correlation therebetween was analyzed.

Accordingly, FIG. 7 illustrates a distribution diagram of T cell receptors with activity specifically induced to an antigen (EGFR-MT-L858R-HVKITDFGR-A*33:03 tetramer). At this time, the analysis of the T cell receptors was performed based on the cells selected in FIG. 2, and V, D, J, and C constituent sequences for the chain of the T cell receptors were analyzed through single cell analysis, and sequencing for a CDR3 sequence was performed based thereon. First, the analyzed T cell receptors had a total of 295 varieties, and among them, the analysis results for the T cell receptor chains up to the top 10 are shown in [Table 1] below, and a combination for TRAV23/DV6 and TRBV18 and a combination for TRAV24 and TRBV19 are shown to have the highest frequency.

**[Table 1]**

| **Rank** | **Frequenc y** | **TCR-alpha** | | | **TCR-beta** | | |
|---|---|---|---|---|---|---|---|
| | | **v_genes** | **j_genes** | **c_genes** | **v_genes** | **j_genes** | **c_genes** |
| 1 | 7 | TRAV23/ DV6 | TRAJ40 | TRAC | TRBV18 | TRBJ 1-5 | TRBC1 |
| 2 | 7 | TRAV24 | TRAJ42 | TRAC | TRBV19 | TRBJ2-1 | TRBC2 |
| 3 | 6 | TRAV16 | TRAJ50 | TRAC | TRBV27 | TRBJ2-1 | TRBC2 |
| 4 | 5 | TRAV8-1 | TRAJ37 | TRAC | TRBV19 | TRBJ2-3 | TRBC2 |
| 5 | 4 | TRAV12- 3 | TRAJ13 | TRAC | TRBV9 | TRBJ2-3 | TRBC2 |
| 6 | 4 | TRAV3 | TRAJ49 | TRAC | TRBV12 -4 | TRBJ1-3 | TRBC1 |
| 7 | 3 | TRAV12- 2 | TRAJ27 | TRAC | TRBV6-1 | TRBJ1-2 | TRBC1 |
| 8 | 3 | TRAV13- 1 | TRAJ42 | TRAC | TRBV24 -1 | TRBJ1-1 | TRBC1 |
| 9 | 3 | TRAV16 | TRAJ54 | TRAC | TRBV7-9 | TRBJ2-1 | TRBC2 |
| 10 | 3 | TRAV27 | TRAJ28 | TRAC | TRBV19 | TRBJ 1-5 | TRBC1 |

Furthermore, based on the analysis results in Table 1 above, the sequences for the generated CDR 3 are shown in Table 2 below, and since all of the sequences were selected based on T cells expressing cytolytic factors, the sequences may include CDR 3 of a TCR alpha chain variable region consisting of amino acid sequences represented by SEQ ID NOs: 1 to 12 and CDR 3 of a TCR beta chain variable region consisting of amino acid sequences represented by SEQ ID NOs: 13 to 24 to have an anticancer effect on EGFR L858R mutant tumors. At this time, the order of [Table 2] is listed according to the expression rate of cytolytic factors (IFN-gamma, and Granzyme-B) (rank 1 represents the highest expression rate).

Furthermore, the TCR having a more effective anticancer effect on EGFR L858R mutant tumors may be a TCR including CDR 3 of a TCR alpha chain variable region consisting of amino acid sequences represented by SEQ ID NOs: 1 to 8 and CDR 3 of a TCR beta chain variable region consisting of amino acid sequences represented by SEQ ID NOs: 13 to 20.

At this time, it was disclosed that the expression level (release ability) of the cytolytic factor was highest in a TCR including CDR 3 of a TCR alpha chain variable region consisting of amino acid sequences represented by SEQ ID NOs: 1 and 2 and CDR 3 of a TCR beta chain variable region consisting of amino acid sequences represented by SEQ ID NOs: 13 and 14. However, the expression levels for the cytolytic factors of a TCR including CDR 3 of a TCR alpha chain variable region consisting of amino acid sequences represented by SEQ ID NOs: 3 to 12 and CDR 3 of a TCR beta chain variable region consisting of amino acid sequences represented by SEQ ID NOs: 15 to 24 were similar.

Thus, the cytolysis effect of a TCR including CDR 3 of a TCR alpha chain variable region consisting of amino acid sequences represented by SEQ ID NOs: 1 to 12 may be the same, and the cytolysis effect of a TCR including CDR 3 of a TCR beta chain variable region consisting of amino acid sequences represented by SEQ ID NOs: 13 to 24 may also be the same.

A TCR with the most effective anticancer effect against EGFR L858R mutant tumors may be a TCR including CDR 3 of a TCR alpha chain variable region consisting of amino acid sequences represented by SEQ ID NOs: 1 and 2 and CDR 3 of a TCR beta chain variable region consisting of amino acid sequences represented by SEQ ID NOs: 13 and 14.

**[Table 2]**

| **Rank** | **Chain** | **CDR3 AA sequence** | **SEQ.** | **CDR3 NA sequence (5' - 3')** | **SEQ.** |
|---|---|---|---|---|---|
| 1 | TRA | CAFIGHGG SQGNLIF | SEQ ID NO: 1 | TGTGCCTTTATAGGCCATG GAGGAAGCCAAGGAAATC TCATCTTT | SEQ ID NO: 25 |
| | TRB | CASSMQG AMSEQFF | SEQ ID NO: 13 | TGTGCCAGTAGTATGCAGG GGGCTATGAGTGAGCAGTT CTTC | SEQ ID NO: 37 |
| 2 | TRA | CAATGTY KYIF | SEQ ID NO: 2 | TGTGCAGCAACAGGAACCT ACAAATACATCTTT | SEQ ID NO: 26 |
| | TRB | CASSPEFA RALDNQPQ HF | SEQ ID NO: 14 | TGTGCCAGCTCACCGGAGT TTGCGAGGGCGCTGGACAA TCAGCCCCAGCATTTT | SEQ ID NO: 38 |
| 3 | TRA | CAYGGGSE KLVF | SEQ ID NO: 3 | TGTGCTTATGGCGGCGGAT CTGAAAAGCTGGTCTTT | SEQ ID NO: 27 |
| | TRB | CASSSATG TQGYTF | SEQ ID NO: 15 | TGTGCCAGCAGCTCGGCCA CAGGGACGCAGGGCTACA CCTTC | SEQ ID NO: 39 |
| 4 | TRA | CALINARL MF | SEQ ID NO: 4 | TGTGCTCTGATTAATGCCA GACTCATGTTT | SEQ ID NO: 28 |
| | TRB | CASSFTNT GELFF | SEQ ID NO: 16 | TGTGCCAGCAGTTTCACCA ACACCGGGGAGCTGTTTTT T | SEQ ID NO: 40 |
| 5 | TRA | CAVNGGS QGNLIF | SEQ ID NO: 5 | TGTGCCGTGAACGGAGGA AGCCAAGGAAATCTCATCT TT | SEQ ID NO: 29 |
| | TRB | CASSMWQ GNGEQYF | SEQ ID NO: 17 | TGTGCCAGTAGTATGTGGC AGGGGAACGGCGAGCAGT ACTTC | SEQ ID NO: 41 |
| 6 | TRA | CAMREGY GGATNKLI F | SEQ ID NO: 6 | TGTGCAATGAGAGAGGGA TATGGTGGTGCTACAAACA AGCTCATCTTT | SEQ ID NO: 30 |
| | TRB | CASSVGPG TTSYNEQF F | SEQ ID NO: 18 | TGTGCCAGCAGCGTAGGCC CAGGGACAACCTCCTACAA TGAGCAGTTCTTC | SEQ ID NO: 42 |
| 7 | TRA | CAYNNGD GGSQGNLI F | SEQ ID NO: 7 | TGTGCTTATAATAACGGGG ATGGAGGAAGCCAAGGAA ATCTCATCTTT | SEQ ID NO: 31 |
| | TRB | CATSRDRS TDTQYF | SEQ ID NO: 19 | TGTGCCACCAGCAGAGACC GTAGCACAGATACGCAGTA TTTT | SEQ ID NO: 43 |
| 8 | TRA | CATDGGSA RQLTF | SEQ ID NO: 8 | TGTGCTACGGACGGGGGTT CTGCAAGGCAACTGACCTT T | SEQ ID NO: 32 |
| | TRB | CASSLGLS GYTF | SEQ ID NO: 20 | TGTGCCAGCAGCTTGGGAT TAAGTGGCTACACCTTC | SEQ ID NO: 44 |
| 9 | TRA | CATLYNTD KLIF | SEQ ID NO: 9 | TGTGCTACGTTATATAACA CCGACAAGCTCATCTTT | SEQ ID NO: 33 |
| | TRB | CASSQSMN TEAFF | SEQ ID NO: 21 | TGTGCCAGCAGCCAGTCAA TGAACACTGAAGCTTTCTT T | SEQ ID NO: 45 |
| 10 | TRA | CAMRGPW RGSSGSAR | SEQ ID NO: 10 | TGTGCAATGAGAGGGCCCT GGAGAGGGTCTTCTGGTTC | SEQ ID NO: 34 |
| | | QLTF | | TGCAAGGCAACTGACCTTT | |
| | TRB | CASRTGLS YEQYF | SEQ ID NO: 22 | TGTGCCAGCAGAACGGGA CTCTCATACGAGCAGTACT TC | SEQ ID NO: 46 |
| 11 | TRA | CALSVRGF KTSYDKVI F | SEQ ID NO: 11 | TGTGCTCTAAGTGTCAGGG GATTCAAAACCTCCTACGA CAAGGTGATATTT | SEQ ID NO: 35 |
| | TRB | CASSFGSA YNEQFF | SEQ ID NO: 23 | TGTGCCAGCAGTTTTGGGT CAGCCTACAATGAGCAGTT CTTC | SEQ ID NO: 47 |
| 12 | TRA | CAVNMMD SSYKLIF | SEQ ID NO: 12 | TGTGCCGTGAACATGATGG ATAGCAGCTATAAATTGAT CTTC | SEQ ID NO: 36 |
| | TRB | CASSFPTA RSNTEAFF | SEQ ID NO: 24 | TGTGCCAGCAGTTTCCCGA CAGCTCGATCGAACACTGA AGCTTTCTTT | SEQ ID NO: 48 |

According to the results above, as the TCR according to an embodiment of the present invention including CDR 3 of the TCR alpha chain variable region consisting of the amino acid sequences represented by SEQ ID NOs: 1 to 12 and CDR 3 of the TCR beta chain variable region consisting of the amino acid sequences represented by SEQ ID NOs: 13 to 24 includes CDR3 expressed at high frequency, the EGFR-L858R mutant antigen (HVKITDFGR-A*33:03) may be more accurately targeted. In addition, as the TCR according to an embodiment of the present invention is very high in the expression of the cytolytic factors, the TCR may have a more effective anticancer response (tumor cytolysis effect) in tumors containing the EGFR-L858R mutant antigen.

### NK cells

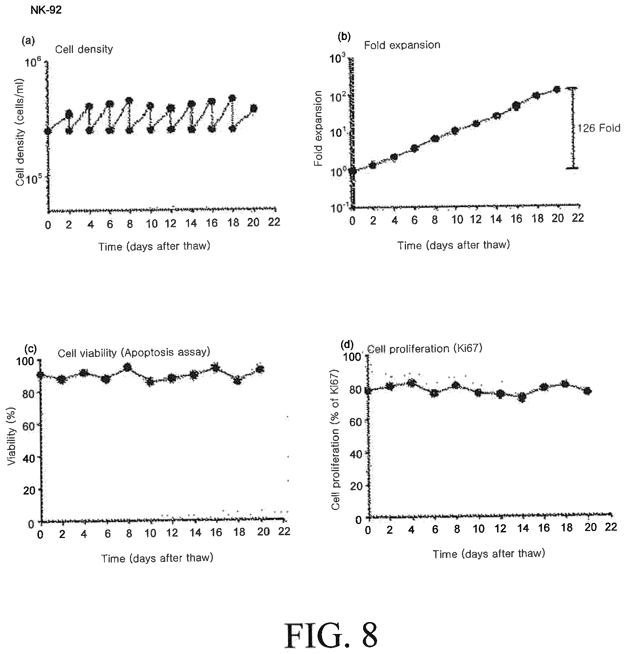

Hereinafter, referring to FIGS. 8 to 14B, NK cells including a TCR and an Fc receptor capable of targeting specific antigens against EGFR mutant tumors will be described.

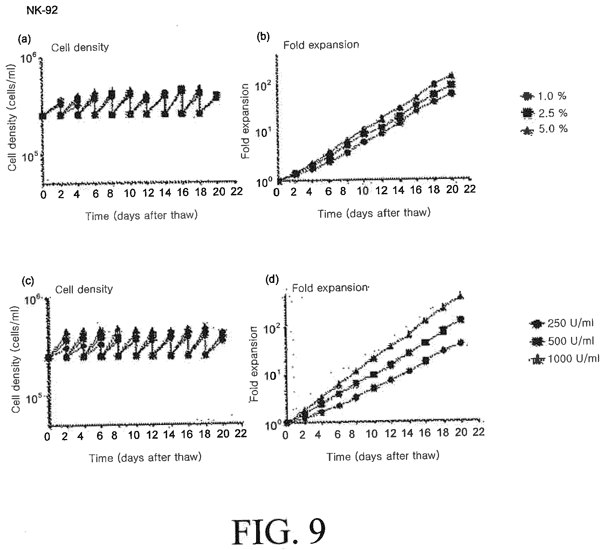

First, referring to FIG. 8, the results for the NK-92 cell line when cultured in a T flask are illustrated. At this time, the NK cells were NK-92 cells and were cultured using X-vivo 10 media or Xuri T cell media, and more specifically, the culture medium was added with 1 to 10% human AB serum, 100 to 2000 U/ml IL-2, 0.1 to 5 mM L-asparagine, 0.1 to 5 mM L-glutamine, and 0.1 to 5 mM L-serine in X-vivo 10 or Xuri T cell media and the capacity thereof is not limited thereto. Furthermore, the NK-92 cells were subcultured at a concentration of 2.5 × 10⁵ cells/ml, cultured in a T flask, and subcultured at 3-day intervals. Furthermore, cell proliferation was confirmed by measuring a cell count using a hematocytometer, cell viability was confirmed using flow cytometry by staining with trypan blue or PI as a cell death marker, and cell proliferation efficiency was confirmed using flow cytometry by staining with KI67, a cell growth factor. The process was performed in the same manner as in FIGS. 8 and 9.

The cell density of NK-92 cells was maintained constant at 1 × 10⁵ to 1 × 10⁶ cells/ml until 22 days of culture due to subculture, and when shown as fold expansion, the number of NK-92 cells increased 126-fold during 22 days of culture. Further, the cell viability was maintained constant at 80 to 100% until 22 days of culture, and the cell proliferation was also maintained constant at 70 to 85% until 22 days of culture.

Thereafter, referring to FIG. 9, results of the proliferation efficiency of NK-92 according to the concentrations of FBS and IL-2 are illustrated.

First, referring to FIGS. 9A and 9B, the proliferation efficiency of NK-92 according to a FBS concentration was the best when FBS was added at 5%. Furthermore, referring to FIGS. 13C and 13D, the proliferation efficiency of NK-92 according to an IL-2 concentration was the best when IL-2 was added at 1000 U/ml. That is, in NK-92 cells, the higher the FBS and IL-2, the higher the proliferation efficiency, and the concentrations of FBS and IL-2 capable of most preferably increasing the proliferation efficiency of NK-92 cells may be 5% and 1000 U/ml, respectively, but are not limited thereto.

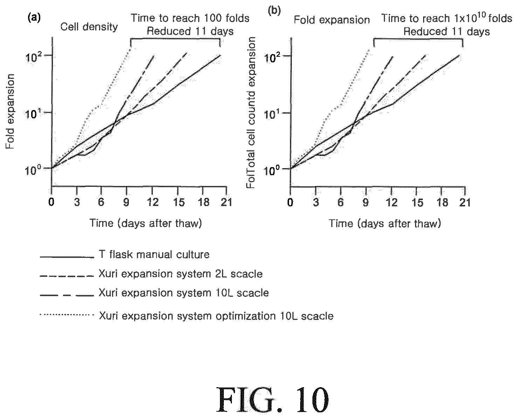

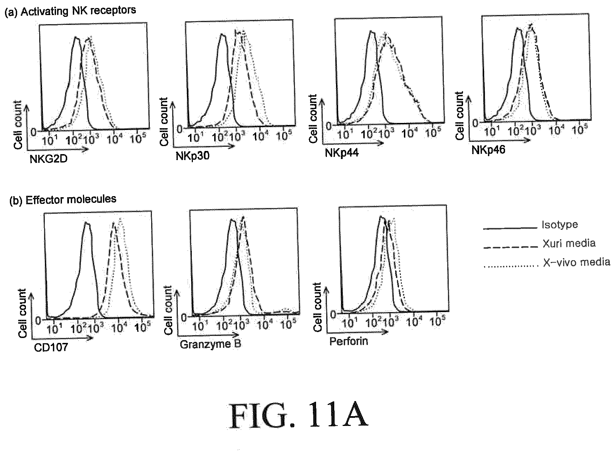

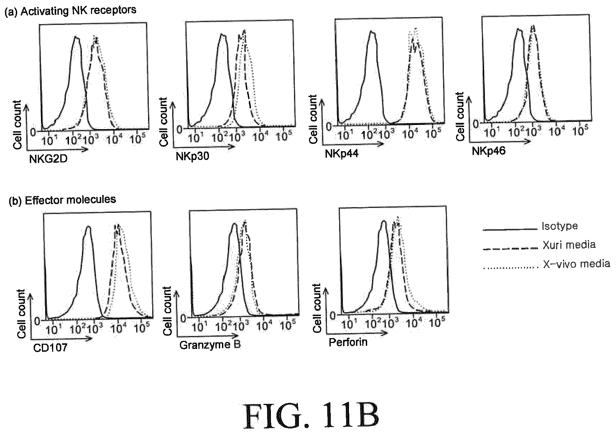

Referring to FIG. 10, the results of the cell proliferation efficiency of a NK-92 cell line according to a culture method are shown. At this time, the NK-92 cells were cultured using Xuri W-25 equipment, which was cell proliferation equipment, for effective proliferation. At this time, the culture medium of NK-92 was the same as the culture medium used in FIGS. 11 to 15 described above, and after 2 L to 10 L of a perfusion bag was installed in the Xuri W-25 equipment, the culture medium was inoculated and stabilized. Furthermore, after the temperature and pH of the culture medium were stabilized, 0.5 to 1.0 × 10⁸ cells/bag of NK-92 cells were inoculated, and a culture environment condition was set to shake at 2 to 2 RPM. In addition, the cell viability, the cell proliferation rate, and the cell count were measured every 12 hours after cell inoculation, and after half of the culture medium was removed through a filter at intervals of 2 days, a new culture medium was inoculated, and finally, the cells were cultured with 1 L of the culture medium in a 2 L perfusion bag and 5 L of the culture medium in a 10 L perfusion bag. Furthermore, when the cell count reached 0.5 to 1.0 × 10¹⁰, NK-92 cells were harvested using a cell harvesting device, Sefia S-2000, and then the cell viability, the cell proliferation rate, and the cell count were measured.

Referring to FIG. 10A, the cell density reached the cell density of 1 × 10² most quickly when cultured on an optimization 10 L scale, and at this time, the time to reach 100 times was shorter by 11 days than a general T flask culture. Furthermore, referring to FIG. 10B, the cell density reached the cell density of 1 × 10¹⁰ most quickly when cultured on an optimization 10 L scale, and at this time, the time to reach 1 × 10¹⁰ was shorter by 11 days than a general T flask culture. Accordingly, when theNK-92 cells were cultured on an optimization 10 L scale in Xuri W-25 equipment, a large number may be obtained in a faster time.

Referring to FIGS. 11A and 11B, results of an NK-92 cell line and activating factors and toxic factors of the NK-92 cell line expressing an Fc receptor according to a culture condition are illustrated. At this time, in order to identify cell active surface factors, cytotoxic factors, and chemokine receptors of the NK-92 cells, the NK-92 cells were stained with each antibody and then confirmed using flow cytometry. More specifically, the cell active surface factors were identified using NKG2D, NKp30, NKp44, and NKp46, and the cytotoxic factors were identified through CD 107, Granzyme B, and Perforin.

Referring to FIG. 11A, results for activating factors and toxic factors of the NK-92 cell line according to culture conditions are illustrated. When NK-92 was cultured in Xuri media, the cell active surface factors NKG2D, NKp30, NKp44, and NKp46 and the cytotoxic factors CD 107, Granzyme B, and Perforin were all expressed. Similarly, when NK-92 was cultured in X-vivo media, the cell active surface factors NKG2D, NKp30, NKp44, and NKp46 and the cytotoxic factors CD 107, Granzyme B, and Perforin were all expressed.

Furthermore, referring to FIG. 11B, results for activating factors and toxic factors of the NK-92 cell line expressing the Fc receptor according to culture conditions are illustrated. When NK-92 expressing the Fc receptor was cultured in Xuri media, the cell active surface factors NKG2D, NKp30, NKp44, and NKp46 and the cytotoxic factors CD 107, Granzyme B, and Perforin were all expressed. Similarly, when NK-92 expressing the Fc receptor was cultured in X-vivo media, the cell active surface factors NKG2D, NKp30, NKp44, and NKp46 and the cytotoxic factors CD 107, Granzyme B, and Perforin were all expressed.

According to the above results, the NK-92 cell line and the NK-92 cell line expressing the Fc receptor may express both cell activity and cytotoxicity when cultured in both Xuri media and X-vivo media.

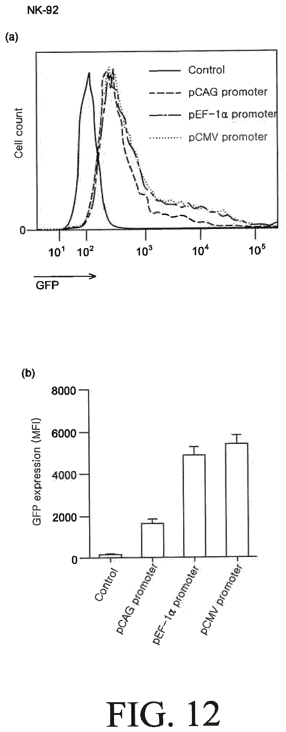

Referring to FIG. 12, results of the GFP expression efficiency of NK-92 cells according to a protein expression promoter are illustrated. At this time, in order to select promoters for the expression of the T cell receptors in NK-92 cells, protein expression according to each promoter was confirmed. More specifically, NK-92 cells were transformed using electroporation and pCAG-GFP, pEF-1α-GFP, and pCMV-GFP plasmids. At this time, the concentration of the plasmid may be 2 ug per 1 × 10⁶ cells, but is not limited thereto. Then, after 24 hours of transduction, the expression of the GFP protein was confirmed using flow cytometry and CAG, EF-1α, CMV, and the like.

As the pCMV promoter shows the highest expression rate for GFP, pCMV may be the most preferable promoter for the expression of the T cell receptors in NK-92 cells.

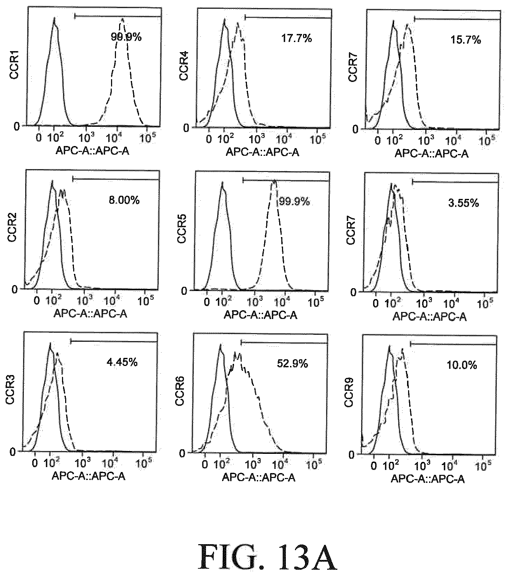

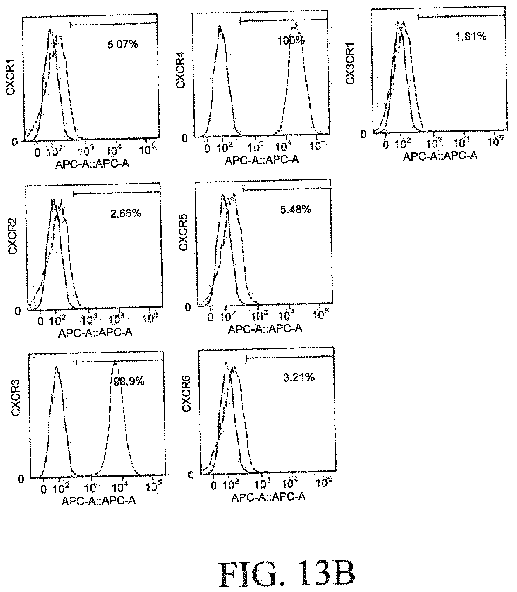

FIGS. 13A and 13B illustrate results of the expression of chemokine receptors in NK-92 cells. At this time, the expression of the chemokine receptors was confirmed by flow cytometry and chemokine receptors CCR1, CCR2, CCR3, CCR4, CCR5, CCR6, CCR7, CCR8, CCR9, CXCR1, CXCR2, CXCR3, CXCR4, CXCR5, CXCR6, and CX3CR1 in the same manner as FIGS. 11A and 11B. First, referring to FIG. 13A, the NK-92 cells cultured according to the above-described culture conditions and methods are shown to express the chemokine receptors CCR1, CCR2, CCR3, CCR4, CCR5, CCR6, CCR7, CCR8, and CCR9.

Furthermore, referring to FIG. 13B, the NK-92 cells are shown to express the chemokine receptors CXCR1, CXCR2, CXCR3, CXCR4, CXCR5, CXCR6, and CX3CR1.

Accordingly, the NK-92 cells cultured according to the above-described culture conditions and methods may activate an acquired immune response by expressing the chemokine receptors.

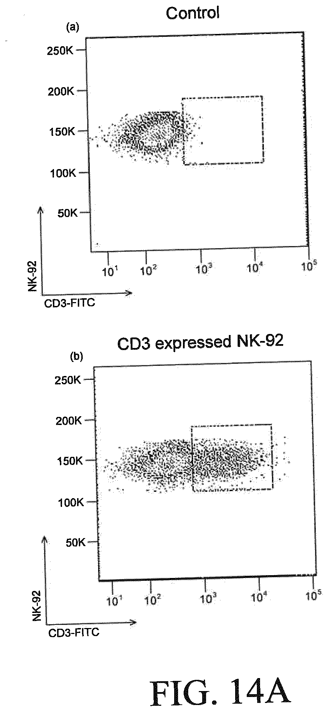

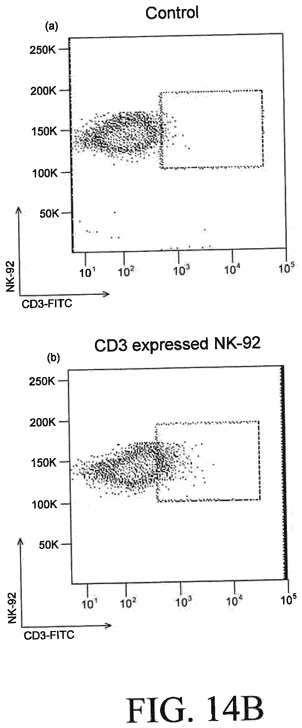

FIGS. 14A and 14B illustrate results of the expression of CD3 molecules and T cell receptor molecules in NK-92 cells. At this time, NK-92 cells were transformed using electroporation and pCMV-CD3γT2AδF2AεP2Aξ and pCMV-TCRαT2Aβ plasmids. At this time, the concentration of the plasmid may be 2 ug per 1 × 10⁶ cells, but is not limited thereto. Then, after 24 hours of transduction, the expression of CD3 molecules and T cell receptor molecules was confirmed using flow cytometry.

First, referring to FIG. 14A, NK-92 cells expressing CD3 molecules account for 28.8% of total NK-92 cells. That is, it may mean that as CD3, an auxiliary protein chain of a T cell antigen receptor, was expressed in NK-92 cells, the TCR was stably transduced into NK-92 cells by the above-described process.

Next, referring to FIG. 14B, NK-92 cells expressing the T cell receptors account for 8.3% of the total NK-92 cells. That is, it may mean that as the T cell antigen receptor was expressed in NK-92 cells, the TCR was stably transduced into NK-92 cells by the above-described process.

Through the above process, the present invention may provide a TCR capable of more effectively targeting solid tumor, particularly lung cancer containing an EGFR mutation, and immune cells, that is, NK cells containing the TCR, thereby improving anticancer effects.

As a result, the immune cells of the present invention may more effectively target lung cancer containing the EGFR mutations by including the above-described TCR, thereby further enhancing the anticancer effect on the targeted cancer cells.

### TCR and NK cells including TCR according to embodiment of present invention

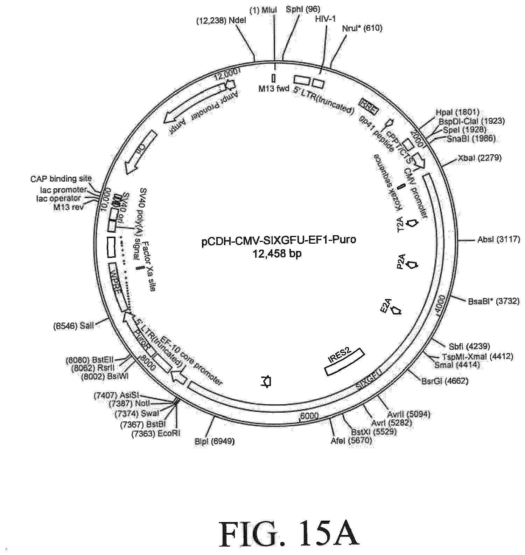

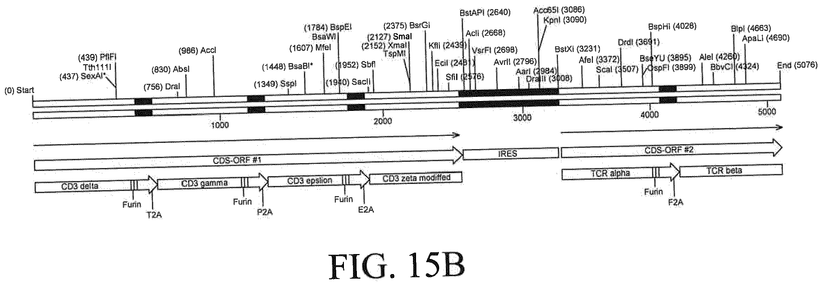

Hereinafter, referring to FIGS. 15A and 15B, the configurations of a TCR according to an embodiment of the present invention (a TCR including CDR 3 of a TCR alpha chain variable region consisting of amino acid sequences represented by SEQ ID NOs: 1 to 12 and CDR 3 of a TCR beta chain variable region consisting of amino acid sequences represented by SEQ ID NOs: 13 to 24) and NK cells including the TCR will be described in detail.

FIG. 15A is a lentiviral vector map for expressing a TCR sequence according to an embodiment of the present invention.

At this time, for transduction into NK-92 cells (expressing a TCR sequence with activity specifically induced by an EGFR-L858R mutant antigen (HVKITDFGR-A*33:03)), a lentiviral vector was used, and vectors that may be used for transduction are not limited to the lentiviral vector, and various vectors that may be used for transduction in the art may all be used.

Lentiviral vector sequence for expression of TCR sequence according to an embodiment of the present invention:

AAATACCGCACAGATGCGTAAGGAGAAAATACCGCATCAGGCGC
CATTCGCCATTCAGGCTGCGCAACTGTTGGGAAGGGCGATCGGTGCGGG
CCTCTTCGCTATTACGCCAGCTGGCGAAAGGGGGATGTGCTGCAAGGCG
ATTAAGTTGGGTAACGCCAGGGTTTTCCCAGTCACGACGTTGTAAAACGA
CGGCCAGTGCCAAGCTG -3' (SEQ ID NO: 50)

Furthermore, the vector may include components of the CD3 molecule and components of TCR alpha and beta.

Accordingly, referring to FIG. 15B, a schematic diagram of nucleic acid sequences of a CD3 molecule and a T cell receptor for expressing a TCR sequence according to an embodiment of the present invention is illustrated.

Components of the nucleic acid sequences of the CD3 molecule and the T cell receptor for expressing the TCR sequence according to an embodiment of the present invention are linked to a Furin + x2A sequence and an IRES sequence for independent expression.

TCR alpha nucleic acid sequence (TRAV23/DV6) for expression of TCR sequence according to an embodiment of the present invention: 5'-ATGGAGACCCTCTTGGGCCTGCTTATCCTTTGGCTGCAGCTGCAATGGGT GAGCAGCAAACAGGAGGTGACGCAGATTCCTGCAGCTCTGAGTGTCCCA GAAGGAGAAAACTTGGTTCTCAACTGCAGTTTCACTGATAGCGCTATTTA CAACCTCCAGTGGTTTAGGCAGGACCCTGGGAAAGGTCTCACATCTCTGT TGCTTATTCAGTCAAGTCAGAGAGAGCAAACAAGTGGAAGACTTAATGC CTCGCTGGATAAATCATCAGGACGTAGTACTTTATACATTGCAGCTTCTC AGCCTGGTGACTCAGCCACCTACCTC**TGTGCAGCAACAGGAACCTACA AATACATCTTT**GGAAGAGGAACCAGCCTTATTGTTCATCCGTATATCCAG AACCCTGACCCTGCCGTGTACCAGCTGAGAGACTCTAAATCCAGTGACAA GTCTGTCTGCCTATTCACCGATTTTGATTCTCAAACAAATGTGTCACAAA GTAAGGATTCTGATGTGTATATCACAGACAAAACTGTGCTAGACATGAG GTCTATGGACTTCAAGAGCAACAGTGCTGTGGCCTGGAGCAACAAATCT GACTTTGCATGTGCAAACGCCTTCAACAACAGCATTATTCCAGAAGACAC CTTCTTCCCCAGCCCAGAAAGTTCCTGTGATGTCAAGCTGGTCGAGAAAA GCTTTGAAACAGATACGAACCTAAACTTTCAAAACCTGTCAGTGATTGGG TTCCGAATCCTCCTCCTGAAAGTGGCCGGGTTTAATCTGCTCATGACGCT GCGGCTGTGGTCCAGC-3' (SEQ ID NO: 51)

TCR beta nucleic acid sequence (TRBV18) for expression of TCR sequence according to an embodiment of the present invention: 5'-ATGAGCATCGGCCTCCTGTGCTGTGCAGCCTTGTCTCTCCTGTGGGCAGG TCCAGTGAATGCTGGTGTCACTCAGACCCCAAAATTCCAGGTCCTGAAGA CAGGACAGAGCATGACACTGCAGTGTGCCCAGGATATGAACCATGAATA CATGTCCTGGTATCGACAAGACCCAGGCATGGGGCTGAGGCTGATTCATT ACTCAGTTGGTGCTGGTATCACTGACCAAGGAGAAGTCCCCAATGGCTAC AATGTCTCCAGATCAACCACAGAGGATTTCCCGCTCAGGCTGCTGTCGGC TGCTCCCTCCCAGACATCTGTGTACTTC**TGTGCCAGCTCACCGGAGTTT GCGAGGGCGCTGGACAATCAGCCCCAGCATTTT**GGCCCAGGCACCCG GCTGACAGTGCTCGAGGACCTGAAAAACGTGTTCCCACCCGAGGTCGCT GTGTTTGAGCCATCAGAAGCAGAGATCTCCCACACCCAAAAGGCCACAC TGGTATGCCTGGCCACAGGCTTCTACCCCGACCACGTGGAGCTGAGCTGG TGGGTGAATGGGAAGGAGGTGCACAGTGGGGTCAGCACAGACCCGCAGC CCCTCAAGGAGCAGCCCGCCCTCAATGACTCCAGATACTGCCTGAGCAGC CGCCTGAGGGTCTCGGCCACCTTCTGGCAGAACCCCCGCAACCACTTCCG CTGTCAAGTCCAGTTCTACGGGCTCTCGGAGAATGACGAGTGGACCCAG GATAGGGCCAAACCCGTCACCCAGATCGTCAGCGCCGAGGCCTGGGGTA GAGCAGACTGTGGCTTCACCTCCGAGTCTTACCAGCAAGGGGTCCTGTCT GCCACCATCCTCTATGAGATCTTGCTAGGGAAGGCCACCTTGTATGCCGT GCTGGTCAGTGCCCTCGTGCTGATGGCCATGGTCAAGAGAAAGGATTCCA GAGGCTAG-3' (SEQ ID NO: 52)

At this time, SEQ ID NOs: 51 and 52 include nucleic acid sequences (SEQ ID NOs: 25 and 37) for the amino acid sequences represented by SEQ ID NOs: 1 and 13, which are TCRs according to an embodiment of the present invention, respectively, and amino acid sequences represented by SEQ ID NOs: 51 and 52 are the same as those represented by SEQ ID NOs: 55 and 56 below (underlined and marked in bold).

TCR alpha amino acid sequence (TRAV23/DV6) for expression of TCR sequence according to an embodiment of the present invention:

METLLGLLILWLQLQWVSSKQEVTQIPAALSVPEGENLVLNCSFTDSAIYNL

QWFRQDPGKGLTSLLLIQSSQREQTSGRLNASLDKSSGRSTLYIAASQPGDSA

TYLCAATGTYKYIFGRGTSLIVHPYIQNPDPAVYQLRDSKSSDKSVCLFTDF

DSQTNVSQSKDSDVYITDKTVLDMRSMDFKSNSAVAWSNKSDFACANAFN

NSIIPEDTFFPSPESSCDVKLVEKSFETDTNLNFQNLSVIGFRILLLKVAGFNLL

MTLRLWSS (SEQ ID NO: 55)

TCR beta amino acid sequence (TRBV18) for expression of TCR sequence according to an embodiment of the present invention:

MSIGLLCCAALSLLWAGPVNAGVTQTPKFQVLKTGQSMTLQCAQDMNHEY
MSWYRQDPGMGLRLIHYSVGAGITDQGEVPNGYNVSRSTTEDFPLRLLSAA
PSQTSVYFCASSPEFARALDNQPQHFGPGTRLTVLEDLKNVFPPEVAVFEPS
EAEISHTQKATLVCLATGFYPDHVELSWWVNGKEVHSGVSTDPQPLKEQPA
LNDSRYCLSSRLRVSATFWQNPRNHFRCQVQFYGLSENDEWTQDRAKPVTQ
IVSAEAWGRADCGFTSESYQQGVLSATILYEILLGKATLYAVLVSALVLMA
MVKRKDSRG-3' (SEQ ID NO: 56)

TCR alpha nucleic acid sequence (TRAV24) for expression of TCR sequence according to an embodiment of the present invention: 5'-ATGGAGACCCTCTTGGGCCTGCTTATCCTTTGGCTGCAGCTGCAATGGGT GAGCAGCAAACAGGAGGTGACGCAGATTCCTGCAGCTCTGAGTGTCCCA GAAGGAGAAAACTTGGTTCTCAACTGCAGTTTCACTGATAGCGCTATTTA CAACCTCCAGTGGTTTAGGCAGGACCCTGGGAAAGGTCTCACATCTCTGT TGCTTATTCAGTCAAGTCAGAGAGAGCAAACAAGTGGAAGACTTAATGC CTCGCTGGATAAATCATCAGGACGTAGTACTTTATACATTGCAGCTTCTC AGCCTGGTGACTCAGCCACCTACCTC**TGTGCCTTTATAGGCCATGGAG GAAGCCAAGGAAATCTCATCTTT**GGAAGAGGAACCAGCCTTATTGTTC ATCCGTATATCCAGAACCCTGACCCTGCCGTGTACCAGCTGAGAGACTCT AAATCCAGTGACAAGTCTGTCTGCCTATTCACCGATTTTGATTCTCAAAC AAATGTGTCACAAAGTAAGGATTCTGATGTGTATATCACAGACAAAACT GTGCTAGACATGAGGTCTATGGACTTCAAGAGCAACAGTGCTGTGGCCTG GAGCAACAAATCTGACTTTGCATGTGCAAACGCCTTCAACAACAGCATTA TTCCAGAAGACACCTTCTTCCCCAGCCCAGAAAGTTCCTGTGATGTCAAG CTGGTCGAGAAAAGCTTTGAAACAGATACGAACCTAAACTTTCAAAACC TGTCAGTGATTGGGTTCCGAATCCTCCTCCTGAAAGTGGCCGGGTTTAAT CTGCTCATGACGCTGCGGCTGTGGTCCAGC-3' (SEQ ID NO: 53)

TCR beta nucleic acid sequence (TRBV19) for expression of TCR sequence according to an embodiment of the present invention: 5'-ATGAGCATCGGCCTCCTGTGCTGTGCAGCCTTGTCTCTCCTGTGGGCAGG TCCAGTGAATGCTGGTGTCACTCAGACCCCAAAATTCCAGGTCCTGAAGA CAGGACAGAGCATGACACTGCAGTGTGCCCAGGATATGAACCATGAATA CATGTCCTGGTATCGACAAGACCCAGGCATGGGGCTGAGGCTGATTCATT ACTCAGTTGGTGCTGGTATCACTGACCAAGGAGAAGTCCCCAATGGCTAC AATGTCTCCAGATCAACCACAGAGGATTTCCCGCTCAGGCTGCTGTCGGC TGCTCCCTCCCAGACATCTGTGTACTTC**TGTGCCAGTAGTATGCAGGGG GCTATGAGTGAGCAGTTCTTC**GGCCCAGGCACCCGGCTGACAGTGCTC GAGGACCTGAAAAACGTGTTCCCACCCGAGGTCGCTGTGTTTGAGCCATC AGAAGCAGAGATCTCCCACACCCAAAAGGCCACACTGGTATGCCTGGCC ACAGGCTTCTACCCCGACCACGTGGAGCTGAGCTGGTGGGTGAATGGGA AGGAGGTGCACAGTGGGGTCAGCACAGACCCGCAGCCCCTCAAGGAGCA GCCCGCCCTCAATGACTCCAGATACTGCCTGAGCAGCCGCCTGAGGGTCT CGGCCACCTTCTGGCAGAACCCCCGCAACCACTTCCGCTGTCAAGTCCAG TTCTACGGGCTCTCGGAGAATGACGAGTGGACCCAGGATAGGGCCAAAC CCGTCACCCAGATCGTCAGCGCCGAGGCCTGGGGTAGAGCAGACTGTGG CTTCACCTCCGAGTCTTACCAGCAAGGGGTCCTGTCTGCCACCATCCTCT ATGAGATCTTGCTAGGGAAGGCCACCTTGTATGCCGTGCTGGTCAGTGCC CTCGTGCTGATGGCCATGGTCAAGAGAAAGGATTCCAGAGGCTAG-3' (SEQ ID NO: 54)

At this time, SEQ ID NOs: 53 and 54 include nucleic acid sequences (SEQ ID NOs: 26 and 38) for the amino acid sequences represented by SEQ ID NOs: 2 and 14, which are TCRs according to an embodiment of the present invention, respectively, and amino acid sequences represented by SEQ ID NOs: 53 and 54 are the same as those represented by SEQ ID NOs: 57 and 58 below (underlined and marked in bold).

TCR alpha amino acid sequence (TRAV24) for expression of TCR sequence according to an embodiment of the present invention:

METLLGLLILWLQLQWVSSKQEVTQIPAALSVPEGENLVLNCSFTDSAIYNL
QWFRQDPGKGLTSLLLIQSSQREQTSGRLNASLDKSSGRSTLYIAASQPGDSA
TYLCAFIGHGGSQGNLIFGRGTSLIVHPYIQNPDPAVYQLRDSKSSDKSVCL
FTDFDSQTNVSQSKDSDVYITDKTVLDMRSMDFKSNSAVAWSNKSDFACAN

AFNNSIIPEDTFFPSPESSCDVKLVEKSFETDTNLNFQNLSVIGFRILLLKVAGF
NLLMTLRLWSS (SEQ ID NO: 57)

TCR beta amino acid sequence (TRBV19) for expression of TCR sequence according to an embodiment of the present invention:

MSIGLLCCAALSLLWAGPVNAGVTQTPKFQVLKTGQSMTLQCAQDMNHEY
MSWYRQDPGMGLRLIHYSVGAGITDQGEVPNGYNVSRSTTEDFPLRLLSAA
PSQTSVYFCASSMQGAMSEQFFGPGTRLTVLEDLKNVFPPEVAVFEPSEAEI
SHTQKATLVCLATGFYPDHVELSWWVNGKEVHSGVSTDPQPLKEQPALNDS
RYCLSSRLRVSATFWQNPRNHFRCQVQFYGLSENDEWTQDRAKPVTQIVSA
EAWGRADCGFTSESYQQGVLSATILYEILLGKATLYAVLVSALVLMAMVKR
KDSRG (SEQ ID NO: 58)

The TCR sequence according to an embodiment of the present invention described above may be expressed and used in various immune cells including not only NK cells, but also T cells, natural killer T cells (NKT), human embryonic stem cells, hematopoietic stem cells (HSC), and induced pluripotent stem cells (iPS). Accordingly, the immune cells including the TCR sequence according to an embodiment of the present invention may have more effective immune response and anticancer effect against various tumor cells including the EGFR-L858R mutant antigen (HVKITDFGR-A*33:03).

### TCR and NK cells including TCR according to embodiment of present invention

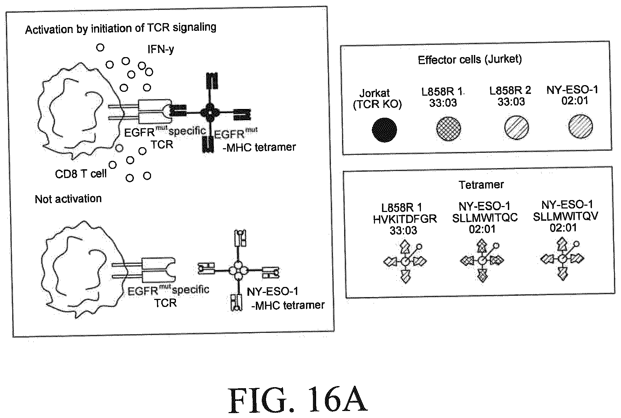

Hereinafter, referring to FIGS. 16A to 18, the anticancer effects of a TCR (including CDR 3 of a TCR alpha chain variable region consisting of amino acid sequences represented by SEQ ID NOs: 1 to 12 and CDR 3 of a TCR beta chain variable region consisting of amino acid sequences represented by SEQ ID NOs: 13 to 24) according to an embodiment of the present invention and NK cells including the TCR will be described in detail.

At this time, the TCR expressed in the NK cells has been used with a TCR including CDR 3 of a TCR alpha chain variable region consisting of amino acid sequences represented by SEQ ID NOs: 1 and 2 and CDR 3 of a TCR beta chain variable region consisting of amino acid sequences represented by SEQ ID NOs: 13 and 14, which is just example. In addition, there is no cytolysis effect from the TCR including CDR 3 of a TCR alpha chain variable region consisting of amino acid sequences represented by SEQ ID NOs: 1 to 12 and CDR 3 of a TCR beta chain variable region consisting of amino acid sequences represented by SEQ ID NOs: 13 to 24, and accordingly, the TCRs may be selectively used.

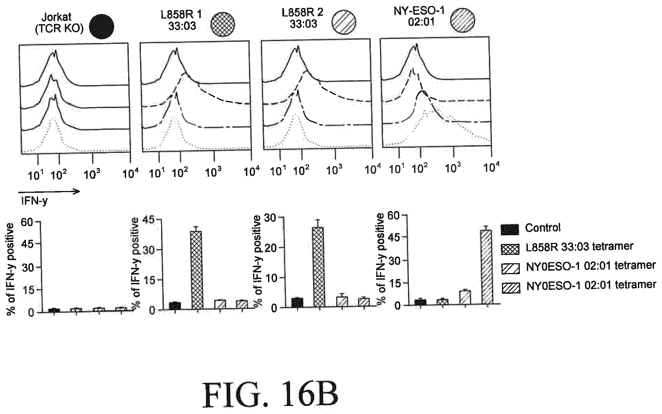

FIGS. 16A and 16B illustrate results of confirming the cell activity of a TCR according to an embodiment of the present invention.

First, referring to FIG. 16A, in Comparative Example 1, knocked-out Jurkat cells were used (control, TCR KO), and in Comparative Example 2, cells (NY-ESO-1 2:01 TCR) expressing a TCR specific to a NY-ESO-1_HLA-A 02:01 antigen in the knocked-out Jurkat cells using lentivirus were used. Furthermore, in Examples 1 (L858R 1 33: 03 TCR) and 2 (L858R 2 33: 03 TCR), cells expressing the TCR according to an embodiment of the present invention in the knocked-out Jurkat cells using lentivirus were used. More specifically, Example 1 (L858R 1 33: 03 TCR) corresponds to cells expressing a TCR based on nucleic acid sequences represented by SEQ ID NOs: 51 and 52 including the amino acid sequences represented by SEQ ID NOs: 1 and 13 (nucleic acid sequences represented by SEQ ID NOs: 25 and 37 for TRAV23/DV6 and TRBV 18). Example 2 (L858R 2 33: 03 TCR) corresponds to cells expressing a TCR based on nucleic acid sequences represented by SEQ ID NOs: 53 and 54 including the amino acid sequences represented by SEQ ID NOs: 2 and 14 (nucleic acid sequences represented by SEQ ID NOs: 26 and 38 for TRAV24 and TRBV 19).

Furthermore, in the case of Examples 1 and 2 (L858R 1 33: 03 TCR and L858R 2 33: 03 TCR), as cytolytic factors such as IFN-gamma may be released by specifically reacting with the EGFR-L858R mutant antigen (HVKITDFGR-A*33:03) tetramer, the expression of IFN-gamma was measured by exposing Comparative Examples 1 and 2 and Examples 1 and 2 to an NY-ESO-1_HLA-A 02:01 antigen tetramer and an EGFR-L858R mutant antigen (HVKITDFGR-A*33:03) tetramer.

Accordingly, referring to FIG. 16B, in the case of Comparative Example 1 (TCR KO Jurkat T cell), as the T cell does not include a TCR capable of recognizing an antigen, there are no IFN-gamma expressing cells (IFN-γ positive cells), and in the case of Comparative Example 2 (NY-ESO-1-TCR Jurkat T cell), as the T cell includes a TCR capable of recognizing only a NY-ESO-1_HLA-A 02:01 antigen, IFN-gamma expressing cells exist only on the NY-ESO-1_HLA-A 02:01 antigen.

Furthermore, in the case of Examples 1 and 2 (L858R 1 33: 03 TCR and L858R 2 33: 03 TCR), as the TCR according to an embodiment of the present invention capable of recognizing the EGFR-L858R mutant antigen (HVKITDFGR-A*33:03) is included, IFN-gamma is expressed by specifically reacting with the EGFR-L858R mutant antigen (HVKITDFGR-A*33:03) tetramer.

That is, it may mean that the TCR according to an embodiment of the present invention is expressed in immune cells and specifically reacts to the EGFR-L858R mutant antigen (HVKITDFGR-A*33:03) to release cytolytic factors (cytokines) such as IFN-gamma.

Furthermore, as the TCR according to an embodiment of the present invention does not induce the expression of IFN-gamma in an NY-ESO-1_HLA-A 02:01 antigen other than the EGFR-L858R mutant antigen (HVKITDFGR-A*33:03), it may mean that a high targeting to the EGFR-L858R mutant antigen (HVKITDFGR-A*33:03) to be targeted by the present invention is possible.

Accordingly, as the TCR according to an embodiment of the present invention can more effectively target tumors including the EGFR-L858R mutant antigen (HVKITDFGR-A*33:03), the immune cells including the TCR according to an embodiment of the present invention may have an enhanced anticancer effect.

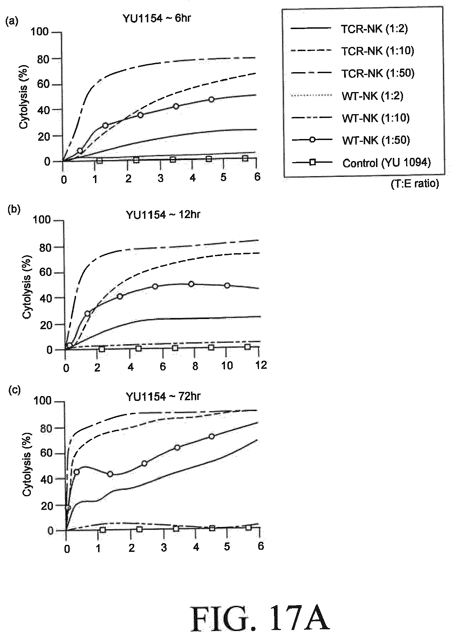

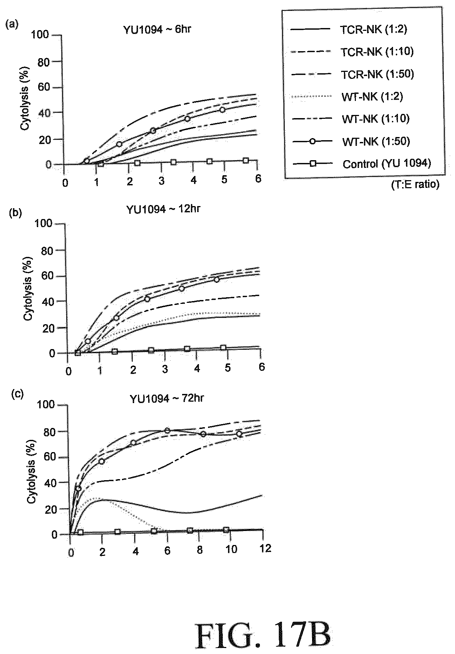

FIGS. 17A and 17B illustrate results of confirming a cytolysis effect on NK cells including a TCR according to an embodiment of the present invention. At this time, in order to confirm the cytolysis effect, a real-time cell analyzer (RTCA) was used, and as tumor cell samples, YU1154 (antigen-specific target cell) and YU1094 (antigen-non-specific target cell, EGFR^{mut HLA11:01}), which were patient-derived tumor cell lines expressing the EGFR-L858R mutant antigen (HVKITDFGR-A*33:03), were used. More specifically, tumor cells (target cells, T), which were the patient-derived tumor cell lines described above, were cultured on a cell culture plate of the analyzer, and then co-cultured with NK-92 cells (effector cells, E) at a certain ratio (T : E ratio), and the cytolysis degree according to a culture time was measured. At this time, the NK-92 cells included wild type (WT) NK-92 cells and NK-92 cells including a TCR (EGFR^{mut HLA33:03}-TCR, DAAN-01) according to an embodiment of the present invention, and the cytolysis degrees therebetween were compared with each other. Furthermore, hereinafter, for convenience of description, it will be described that the NK-92 cells including the TCR according to an embodiment of the present invention are named Examples, and wild type (WT) NK-92 cells are named Comparative Examples.

First, referring to FIG. 17A, the cytolysis effect on YU1154 (antigen-specific target cells) is illustrated, NK cells (TCR-NK) including the TCR according to an embodiment of the present invention, that is, Examples have a higher cytolysis effect than Comparative Example (wild type NK cells).

More specifically, referring to FIG. 17A (a), when co-cultured at a ratio of 10 and 50 (TCR-NK 1 : 10, 1 : 50) with target cell (tumor cell) 1, Example of the present invention has a cytolysis effect of about 60% or more for 6 hours. In contrast, all of Comparative Examples show a cytolysis effect of 50% or less regardless of the rate for the target cells (tumor cells).

Furthermore, referring to FIG. 17A (b), when co-cultured at a ratio of 10 and 50 (TCR-NK 1: 10, 1 : 50) with target cell (tumor cell) 1, Example of the present invention has a cytolysis effect of about 70 % or more for 12 hours. In contrast, all of Comparative Examples show a cytolysis effect of 50% or less regardless of the rate for the target cells (tumor cells).

Furthermore, referring to FIG. 17A (c), when co-cultured at a ratio of 10 and 50 (TCR-NK 1 : 10, 1 : 50) with target cell (tumor cell) 1, Example of the present invention has a cytolysis effect of about 85 % or more for 72 hours. On the other hand, except for when co-cultured at a ratio of 50 (WT-NK 1 : 50) to target cells (tumor cells) 1, all of Comparative Examples have a cytolysis effect of 10% or less, and even when co-cultured at a ratio of 50 (WT-NK 1 : 50) to target cells (tumor cells) 1, Comparative Examples have only a cytolysis effect of about 80%, which is lower than that of Examples of the present invention described above.

As a result, the NK cells including the TCR according to an embodiment of the present invention may more effectively induce cytolysis in the EGFR-L858R mutant antigen (HVKITDFGR-A*33:03), thereby exhibiting an enhanced anticancer effect on tumors containing the EGFR-L858R mutant antigen (HVKITDFGR-A*33:03).

Next, referring to FIG. 17B, a cytolysis effect on YU1094 (antigen non-specific target cells) is illustrated, NK cells (TCR-NK) including the TCR according to an embodiment of the present invention, that is, Examples have a higher cytolysis effect than Comparative Example (wild type NK cells).

More specifically, referring to FIG. 17B (a), when co-cultured at a ratio of 10 and 50 (TCR-NK 1 : 10, 1 : 50) with target cell (tumor cell) 1, Example of the present invention has a cytolysis effect of about 40 % or more for 6 hours. In contrast, all of Comparative Examples show a cytolysis effect of 40 % or less regardless of the rate for the target cells (tumor cells).

Furthermore, referring to FIG. 17B (b), when co-cultured at a ratio of 10 and 50 (TCR-NK 1 : 10, 1 : 50) with target cell (tumor cell) 1, Example of the present invention has a cytolysis effect of about 50 % or more for 12 hours. On the other hand, except for when co-cultured at a ratio of 50 (WT-NK 1 : 50) to target cells (tumor cells) 1, all of Comparative Examples have a cytolysis effect of 40 % or less, and even when co-cultured at a ratio of 50 (WT-NK 1 :50) to target cells (tumor cells) 1, Comparative Examples have only a cytolysis effect of about 5 %, which is lower than that of Examples of the present invention described above.

Furthermore, referring to FIG. 17B (c), when co-cultured at a ratio of 10 and 50 (TCR-NK 1 : 10, 1 : 50) with target cell (tumor cell) 1, Example of the present invention has a cytolysis effect of about 70 % or more for 72 hours. On the other hand, all of Comparative Examples have a lower cytolysis effect than Examples of the present invention (TCR-NK 1 : 10, 1 : 50) described above.

As a result, the NK cells including the TCR according to an embodiment of the present invention may more effectively induce cytolysis in the EGFR-L858R mutant antigen (HVKITDFGR-A*33:03), thereby exhibiting an enhanced anticancer effect on tumors containing the EGFR-L858R mutant antigen (HVKITDFGR-A*33:03).

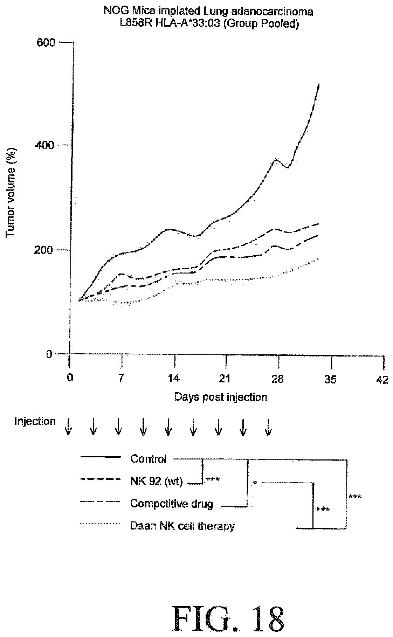

FIG. 18 is a result of confirming a tumor suppression effect of NK cells including the TCR according to an embodiment of the present invention. At this time, the tumor suppression effect was confirmed in a patient-derived tumor animal model (NOG mice implanted lung adenocarcinoma) induced to express the EGFR-L858R mutant antigen (HVKITDFGR-A*33:03). In the tumor animal model, the patient-derived tumor tissue including the EGFR-L858R mutant antigen (HVKITDFGR-A*33:03) was implanted and then bred for 4 to 5 months so that the size of the tumor became 100 to 150 mm³ or more, and then injected with NK-92 cells containing the TCR according to an embodiment of the present invention when reached the target size. Furthermore, the injection of NK cells containing the TCR according to an embodiment of the present invention was performed twice/week for a total of 4 weeks, and the injection amount was selected from the range of 1 × 10⁷ to 5 × 10⁷ per injection. The injection dose of the NK cells including the TCR according to an embodiment of the present invention may be variously selected and used according to a usage of the cell therapy. Also, as Comparative Example, wild-type NK-92 cells and a competitive drug, that is, CD16-NK-92 cells were injected and used, respectively.

Referring to FIG. 18, when injecting NK cells (Daan NK cell therapy) containing the TCR according to an embodiment of the present invention, the tumor volume of the animal model was about 200% or less, which was lower than that in treatment with the competitive drug and the wild-type NK cells.

That is, it may mean that the NK cells containing the TCR according to an embodiment of the present invention are more effective in suppressing tumors than injection of conventional anticancer drugs and general immune cells. In particular, it may mean that as the NK cells containing the TCR according to an embodiment of the present invention have a statistically significant difference compared to injection of normal immune cells (p < 0.001), the target anti-cancer (immune) effect is remarkably improved in tumors containing the EGFR-L858R mutant antigen (HVKITDFGR-A*33:03) (* = p < 0.05, *** = p < 0.001).

Although the embodiments of the present invention have been described in detail with reference to the accompanying drawings, the present invention is not limited thereto and may be embodied in many different forms without departing from the technical concept of the present invention. Therefore, the embodiments of the present invention are provided for illustrative purposes only but not intended to limit the technical concept of the present invention. The scope of the technical concept of the present invention is not limited thereto. Therefore, it should be appreciated that the aforementioned embodiments are illustrative in all aspects and are not restricted. The protective scope of the present invention should be construed on the basis of the appended claims, and all the technical spirits in the equivalent scope thereof should be construed as falling within the scope of the present invention.

## Claims

1. A T cell receptor comprising:
(i) a complementarity determining region (CDR) 3 of a T cell receptor (TCR) alpha chain variable region including an amino acid sequence CAFIGHGGSQGNLIF (SEQ ID NO: 1) or a variant thereof, and
a CDR 3 of a TCR beta chain variable region including an amino acid sequence CASSMQGAMSEQFF (SEQ ID NO: 13) or a variant thereof,
(ii) a CDR 3 of a TCR alpha chain variable region including an amino acid sequence CAATGTYKYIF (SEQ ID NO: 2) or a variant thereof, and
a CDR 3 of a TCR beta chain variable region including an amino acid sequence CASSPEFARALDNQPQHF (SEQ ID NO: 14) or a variant thereof,
(iii) a CDR 3 of a TCR alpha chain variable region including an amino acid sequence CAYGGGSEKLVF (SEQ ID NO: 3) or a variant thereof, and
a CDR 3 of a TCR beta chain variable region including an amino acid sequence CASSSATGTQGYTF (SEQ ID NO: 15) or a variant thereof,
(iv) a CDR 3 of a TCR alpha chain variable region including an amino acid sequence CALINARLMF (SEQ ID NO: 4) or a variant thereof, and
a CDR 3 of a TCR beta chain variable region including an amino acid sequence CASSFTNTGELFF (SEQ ID NO: 16) or a variant thereof,
(v) a CDR 3 of a TCR alpha chain variable region including an amino acid sequence CAVNGGSQGNLIF (SEQ ID NO: 5) or a variant thereof, and
a CDR 3 of a TCR beta chain variable region including an amino acid sequence CASSMWQGNGEQYF (SEQ ID NO: 17) or a variant thereof,
(vi) a CDR 3 of a TCR alpha chain variable region including an amino acid sequence CAMREGYGGATNKLIF (SEQ ID NO: 6) or a variant thereof, and
a CDR 3 of a TCR beta chain variable region including an amino acid sequence CASSVGPGTTSYNEQFF (SEQ ID NO: 18) or a variant thereof,
(vii) a CDR 3 of a TCR alpha chain variable region including an amino acid sequence CAYNNGDGGSQGNLIF (SEQ ID NO: 7) or a variant thereof, and
a CDR 3 of a TCR beta chain variable region including an amino acid sequence CATSRDRSTDTQYF (SEQ ID NO: 19) or a variant thereof,
(viii) a CDR 3 of a TCR alpha chain variable region including an amino acid sequence CATDGGSARQLTF (SEQ ID NO: 8) or a variant thereof, and
a CDR 3 of a TCR beta chain variable region including an amino acid sequence CASSLGLSGYTF (SEQ ID NO: 20) or a variant thereof,
(ix) a CDR 3 of a TCR alpha chain variable region including an amino acid sequence CATLYNTDKLIF (SEQ ID NO: 9) or a variant thereof, and
a CDR3 of a TCR beta chain variable region including an amino acid sequence CASSQSMNTEAFF (SEQ ID NO: 21) or a variant thereof,
(x) a CDR 3 of a TCR alpha chain variable region including an amino acid sequence CAMRGPWRGSSGSARQLTF (SEQ ID NO: 10) or a variant thereof, and
a CDR 3 of a TCR beta chain variable region including an amino acid sequence CASRTGLSYEQYF (SEQ ID NO: 22) or a variant thereof,
(xi) a CDR 3 of a TCR alpha chain variable region including an amino acid sequence CALSVRGFKTSYDKVIF (SEQ ID NO: 11) or a variant thereof, and
a CDR 3 of a TCR beta chain variable region including an amino acid sequence CASSFGSAYNEQFF (SEQ ID NO: 23) or a variant thereof, or
(xii) a CDR 3 of a TCR alpha chain variable region including an amino acid sequence CAVNMMDSSYKLIF (SEQ ID NO: 12) or a variant thereof, and
a CDR 3 of a TCR beta chain variable region including an amino acid sequence CASSFPTARSNTEAFF (SEQ ID NO: 24) or a variant thereof.

2. The T cell receptor of claim 1, wherein the T cell receptor is able to bind to an epitope included in an amino acid sequence represented by HVKITDFGR (SEQ ID NO: 49) or an MHC-binding form thereof.

3. The T cell receptor of claim 2, wherein the epitope has a binding affinity with at least one of HLA-A*33:03 and HLA-A*31:01.

4. The T cell receptor of claim 1, wherein the T cell receptor targets an EGFR L858R mutation.

5. The T cell receptor of claim 1, wherein the TCR alpha chain variable region consists of an amino acid sequence represented by SEQ ID NO: 55, and
the SEQ ID NO: 55 includes an amino acid sequence represented by SEQ ID NO: 1.

6. The T cell receptor of claim 1, wherein the TCR alpha chain variable region consists of an amino acid sequence represented by SEQ ID NO: 57, and
the SEQ ID NO: 57 includes an amino acid sequence represented by SEQ ID NO: 2.

7. The T cell receptor of claim 1, wherein the TCR beta chain variable region consists of an amino acid sequence represented by SEQ ID NO: 56, and
the SEQ ID NO: 56 includes an amino acid sequence represented by SEQ ID NO: 13.

8. The T cell receptor of claim 1, wherein the TCR beta chain variable region consists of an amino acid sequence represented by SEQ ID NO: 58, and
the SEQ ID NO: 58 includes an amino acid sequence represented by SEQ ID NO: 14.

9. The T cell receptor of claim 1, wherein the T cell receptor is a single chain type.

10. The T cell receptor of claim 2, wherein the TCR alpha chain variable region and the TCR beta chain variable region are linked to each other by a linker sequence.

11. A nucleic acid encoding the T cell receptor according to any one of claims 1 to 10.

12. The nucleic acid of claim 11, wherein the nucleic acid includes at least one nucleic acid sequence represented by SEQ ID NOs: 51 to 54; or
a nucleic acid sequence having at least 80% or more identity with at least one nucleic acid sequence represented by SEQ ID NOs: 51 to 54.

13. The nucleic acid of claim 12, wherein the SEQ ID NOs: 51 and 53 are nucleic acid sequences encoding a TCR alpha chain variable region.

14. The nucleic acid of claim 12, wherein the SEQ ID NOs: 52 and 54 are nucleic acid sequences encoding a TCR beta chain variable region.

15. The nucleic acid of claim 11, further comprising:
Furin, 2A, and IRES sequences.

16. A vector comprising the nucleic acid according to any one of claims 11 to 15.

17. The vector of claim 16, wherein the vector is an expression vector.

18. The vector of claim 17, wherein the expression vector is a lentiviral vector.

19. The vector of claim 16, wherein the vector includes a nucleic acid sequence represented by SEQ ID NO: 50; or
a nucleic acid sequence having at least 80% or more identity with the nucleic acid sequence represented by SEQ ID NO: 50.

20. An immune cell comprising the T cell receptor according to any one of claims 1 to 10.

21. The immune cell of claim 20, wherein the immune cell is an NK-92 cell.

22. A cell therapy comprising an immune cell including the T cell receptor according to any one of claims 1 to 10.

23. The cell therapy of claim 22, further comprising:
an effector T cell.

24. The cell therapy of claim 22, wherein the cell therapy is a solid tumor therapy.
